# EUROPEAN PATENT SPECIFICATION

(11) **EP 1 833 506 B1**
(45) Date of publication and mention of the grant of the patent: **26.08.2015**
(21) Application number: 05855898.2
(22) Date of filing: 29.12.2005
(51) Int. Cl.: A61K 39/00, A61P 35/00

(54) **USE OF COMPOSITIONS COMPRISING VARIOUS TUMOR-ASSOCIATED ANTIGENS AS ANTI-CANCER VACCINES**
VERWENDUNG VON ZUBEREITUNGEN, DIE VERSCHIEDENE TUMOR-ASSOZIIERTE ANTIGENE ALS IMPFSTOFFE GEGEN KREBS ENTHALTEN
UTILISATION DE COMPOSITIONS COMPRENANT DES COMBINAISONS D'ANTIGENES ASSOCIÉS À UNE TUMEUR COMME VACCIN ANTI-CANCEREUX

(30) Priority: 29.12.2004 US 640598 P
(43) Date of publication of application: 19.09.2007
(73) Proprietor: Mannkind Corporation, Valencia, California 91355 (US)
(72) Inventor: CHIANG, Chih-Sheng, Chatsworth, California 91311 (US); SIMARD, John, J.L., West Vancouver, British Columbia V7V 3C1 (CA); DIAMOND, David, C., West Hills, California 91304 (US); BOT, Adrian, Ion, Valencia, California 91354 (US); LIU, Xiping, Temple City, California 91780 (US)
(74) Representative: Neuefeind, Regina
(86) International application number: PCT/US2005/047407
(87) International publication number: WO 2006/071983

(56) References cited:
- EP-A2- 1 595 548
- WO-A-01/85932
- WO-A-2004/112825
- WO-A-2006/002114
- WO-A-2006/014579
- US-A1- 2004 132 088
- REYNOLDS S R ET AL: "Identification of HLA-A*03, A*11 and B*07-restricted melanoma-associated peptides that are immunogenic in vivo by vaccine-induced immune response (VIIR) analysis" JOURNAL OF IMMUNOLOGICAL METHODS, ELSEVIER SCIENCE PUBLISHERS B.V.,AMSTERDAM, NL, vol. 244, no. 1-2, 20 October 2000 (2000-10-20), pages 59-67, XP004218446 ISSN: 0022-1759
- SMITH S G ET AL: "Human dendritic cells genetically engineered to express a melanoma polyepitope DNA vaccine induce multiple cytotoxic T-cell responses" CLINICAL CANCER RESEARCH, THE AMERICAN ASSOCIATION FOR CANCER RESEARCH, US, vol. 7, no. 12, December 2001 (2001-12), pages 4253-4261, XP002219398 ISSN: 1078-0432
- CHANG S S ET AL: "FIVE DIFFERENT ANTI-PROSTATE-SPECIFIC MEMBRANE ANTIGEN (PSMA) ANTIBODIES CONFIRM PSMA EXPRESSION IN TUMOR-ASSOCIATED NEOVASCULATURE" CANCER RESEARCH, AMERICAN ASSOCIATION FOR CANCER RESEARCH, BALTIMORE, MD, US, vol. 59, 1 July 1999 (1999-07-01), pages 3192-3198, XP000941661 ISSN: 0008-5472
- MOINGEON P: "Cancer vaccines" VACCINE, BUTTERWORTH SCIENTIFIC. GUILDFORD, GB, vol. 19, no. 11-12, 8 December 2001 (2001-12-08), pages 1305-1326, XP004313943 ISSN: 0264-410X
- KESSLER J H ET AL: "Efficient identification of novel HLA-A(*)0201-presented cytotoxic T lymphocyte epitopes in the widely expressed tumor antigen PRAME by proteasome-mediated digestion analysis" JOURNAL OF EXPERIMENTAL MEDICINE, TOKYO, JP, vol. 193, no. 1, 1 January 2001 (2001-01-01), pages 73-88, XP002339970 ISSN: 0022-1007
- MATSUSHITA M ET AL: "PREFERENTIALLY EXPRESSED ANTIGEN OF MELANOMA (PRAME) IN THE DEVELOPMENT OF DIAGNOSTIC AND THERAPEUTIC METHODS FOR HEMATOLOGICAL MALIGNANCIES" LEUKEMIA AND LYMPHOMA, HARWOOD ACADEMIC PUBLISHERS, CHUR, CH, vol. 44, no. 3, March 2003 (2003-03), pages 439-444, XP009040231 ISSN: 1042-8194
- BOT ADRIAN I ET AL: "A novel class of biotherapeutics co-targeting cancer cells and the associated tumor neovasculature" JOURNAL OF IMMUNOTHERAPY, vol. 28, no. 6, November 2005 (2005-11), page 637, XP009067901 & 20TH ANNUAL SCIENTIFIC MEETING OF THE INTERNATIONAL-SOCIETY-FOR-BIOLO GICAL-THERAPY-OF-CANCER; ALEXANDRIA, VA, USA; NOVEMBER 10 -13, 2005 ISSN: 1524-9557 -& DATABASE INTERNET [Online] 13 November 2005 (2005-11-13), XP002385468 retrieved from HTTP://WWW.ISBTC.ORG/MEETINGS/AM05/PRESENT ATIONS/BOT.PDF
- TAJEDDINE N ET AL: "Tumor-associated antigen preferentially expressed antigen of melanoma (PRAME) induces caspase-independent cell death in vitro and reduces tumorigenicity in vivo" CANCER RESEARCH, AMERICAN ASSOCIATION FOR CANCER RESEARCH, BALTIMORE, MD, US, vol. 65, no. 16, August 2005 (2005-08), pages 7348-7355, XP002372786 ISSN: 0008-5472

## Description

### Background of the Invention

### Field of the Invention

Disclosed herein are methods and compositions for inducing an immune response against various combinations of tumor-associated antigens, which can promote effective immunologic intervention in pathogenic processes.

### Description of the Related Art

The American Cancer Society has estimated that over one million people get cancer each year, and that approximately one out of every two American men and one out of every three American women will have some type of cancer at some point during their lifetime.

Cancer generally develops when cells in a part of the body begin to grow out of control. Although there are many kinds of cancer, they typically begin with out-of-control growth of abnormal cells.

Normal body cells grow, divide, and die in an orderly fashion. Cancer cells are different in that they continue to grow and divide. Instead of dying, they outlive normal cells and continue to form new abnormal cells.

Usual treatment options for cancer include surgery, radiation therapy, and chemotherapy. A fourth branch of treatment is developing, which is referred to as immunotherapy. Immunotherapies attempt to help the immune system recognize cancer cells, and/or to strengthen a response against cancer cells in order to destroy the cancer. Immunotherapies include active and passive immunotherapies. Active immunotherapies attempt to stimulate the body's own immune system to fight the disease. Passive immunotherapies generally do not rely on the body to attack the disease; instead, they use immune system components (such as antibodies) created outside of the body.

A continuing need exists for additional treatment options.

Chang et al. (1999) Cancer Research 59: 3192-3198 discloses that PSMA is expressed in nonprostatic tumor neovasculature.

### Summary of the Invention

Embodiments of the invention disclosed herein are directed to the use of effective combinations of tumor-associated antigens (TuAAs) for the immunotherapy of patients with pancreatic cancer. In some embodiments, the TuAAs are antigens expressed by the cancer cell itself. In some embodiments, the TuAAs are antigens associated with non-cancerous components of the tumor, such as tumor-associated neovasculature or other stroma. In some embodiments, the combinations further include a tumor growth factor and/or a signal transduction protein. Immunogenic compositions for inducing an immune response to these combinations of antigens are disclosed.

Some embodiments relate to the use of a composition comprising, for example, PRAME and PSMA and optionally at least one other tumor-associated antigen in the preparation of a medicament for the treatment of pancreatic cancer. The composition can further include an antigen associated with tumor neovasculature, a growth factor, or a signal transduction protein. The at least one other tumor-associated antigen can be, for example, SSX-2, NY-ESO-1, a MAGE protein, MAGE-3, Melan-A, PLK1, VEGF-A, VEGFR2, Tie-2, and the like, or subsets thereof. The composition can further include at least one antigen associated with tumor neovasculature. The antigen associated with tumor neovasculature can be, for example, VEGFR2 and/or Tie-2. The growth factor can be VEGF-A, for example. The signal transduction protein can be, for example, PLK1. In a preferred embodiment, the medicament can induce a cytolytic T cell response. In some embodiments, the medicament is for use in combination with at least one additional medicament comprising a tumor-associated antigen.

Other embodiments relate to immunogenic compositions for use in the treatment of pancreatic cancer. The compositions can include, for example, individually or in combination, 1) whole antigens, 2) fragments of antigens, 3) epitope clusters derived from antigens, 4) epitopes derived from antigens, or 5) nucleic acids encoding any of 1 to 4; wherein the antigens are PRAME and PSMA and optionally at least one other tumor-associated antigen. In a preferred embodiment, the at least one other tumor-associated antigen can be selected from SSX-2, NY-ESO-1, a MAGE protein, MAGE-3, Melan-A, VEGF-A, PLK1, VEGFR2, Tie-2, and the like, or subsets thereof. In some embodiments, the composition comprises PRAME, PSMA and NY-ESO-1 and/or SSX-2. In some embodiments, the compositions can further include a neovasculature antigen or other stromal cell antigen. The antigen associated with tumor neovasculature can be VEGFR2 and/or Tie-2. In some embodiments, the compositions can also include an extra-cellular factor. In some embodiments, the compositions further include a non-target antigen. In other embodiments, the compositions can include a means for inducing immunity to a factor that promotes the growth, survival, invasiveness, or metastasis of a tumor. The compositions can also include a means for inducing bystander help for the tumor-associated antigens. In some embodiments, the compositions can include a means for causing inflammation in a tumor lesion. The compositions can further include a growth factor and/or a signal transduction protein. The growth factor can be, for example, VEGF-A. The signal transduction factor can be, for example, PLK1.

In some embodiments, the use can further include the use of a stromal cell antigen. In some embodiments, the use further includes the use of an extra-cellular factor antigen. The extra-cellular factor can be, for example, an autocrine factor, a paracrine factor, a growth factor, chorionic gonadatropin, gastrin, an NF-kB activating factor, VEGF-A CXCL1, CXCL8, CCL2 and the like. In some embodiments, the use can further include the use of means for inducing immunity to a factor that promotes the growth, survival, invasiveness, or metastasis of a tumor.

In another embodiment, the use can include the use of a non-self antigen, such as a B cell epitope or a Th epitope.

In some embodiments, the use can be combined with a treatment such as, for example, chemotherapy, radiotherapy, biotherapy, passive immunotherapy, antibody therapy, surgery, and the like.

### Brief Description of the Drawings

Figure 1 is a timeline depicting the schedule of immunization with two plasmids (pCBP expressing SSX2 41-49 and pSEM expressing Melan A).
Figure 2 is a bar graph that shows CTL activity obtained using the protocol in Figure 1.
Figure 3 is a timeline depicting the schedule of immunization of an entrain-and-amplify immunization protocol using plasmids and peptides representing two epitopes.
Figure 4 is a table showing *in vivo* clearance of epitope-pulsed cells in mice immunized according to the protocol of Figure 3.
Figures 5A and 5B are timelines depicting preferred immunization protocols for inducing strong multivalent responses. Figure 5A shows the use of peptides for boosting restores multivalent immune responses even if plasmids and peptides are used as mixtures. Figure 5B shows segregation of plasmid and peptide components allows induction of multivalent immune responses.

### Detailed Description of the Preferred Embodiment

The frequency of expression of many tumor-associated antigens (TuAAs) in various types of cancers is known. However, the frequency of appearance of some antigens, and especially certain combinations of TuAAs, in various types of cancers has not been reported. Accurate measurement of the presence of TuAAs in tumor tissues aids in determining which TuAAs will be useful for the treatment of a particular type of cancer.

Many attempts to develop active immunotherapies for cancer have utilized a single antigen. This can be problematic for two distinct reasons. Firstly, the expression of any particular TuAA in cancer can be mosaic with the antigen expression ranging from high in some cells within a tumor mass to completely absent in others. Moreover, the TuAA may be expressed in some lesions but not others. By directing an immune response against more than a single antigen, if properly selected, the number of tumor cells that can be recognized is maximized. Secondly, some tumors lose expression of a TuAA following immunization, giving rise to a resistant population. If the immune response is directed against more than one TuAA it becomes much more difficult for a resistant tumor to arise because it must then simultaneously lose expression of each of the antigens in order to escape. Thus, in treating cancer with immunotherapy, it can be advantageous to use a combination of TuAAs both due to more complete coverage of the population of tumor cells, and because there will be less chance of tumor escape through loss of expression of the TuAAs. In preferred embodiments, this technique is employed when the tumor is positive for two, three, or more of the TuAAs of the combination used.

Multivalent attack can offer another advantage in increasing the sensitivity of the tumor to attack. If more than a single antigen on a tumor cell is targeted, the effective concentration of anti-tumor agent is increased. In addition, attack on stroma associated with the tumor, such as vasculature, can increase the accessibility of the tumor cells to the agent(s) targeting them. Thus, even an antigen that is also expressed on some normal tissue can receive greater consideration as a target antigen, if the other antigens to be targeted in a multivalent attack are not also expressed by that tissue.

Some embodiments include the use of at least two different compositions and can involve several compositions to be administered together and/or at different times. Thus, embodiments of the invention include sets and subsets of immunogenic compositions and individual doses thereof. Multivalency can be achieved using compositions comprising multivalent immunogens, combinations of monovalent immunogens, coordinated use of compositions comprising one or more monovalent immunogens or various combinations thereof. Multiple compositions, manufactured for use in a particular treatment regimen or protocol according to such methods, define an immunotherapeutic product. In some embodiments all or a subset of the compositions of the product are packaged together in a kit.

### Definitions

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell co-stimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; likewise, the mature epitope can be provided in its ultimate form that can bind in the MHC binding cleft to be recognized by TCR.

MHC EPITOPE - a polypeptide having a known or predicted binding affinity for a mammalian class I or class II major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provided in U.S. Patent Application Pub. No. 2003-0220239 A1, Pub. No. 2005-0221440 A1, Pub. No. 2005-0142144 A1 and Pub. No. 2004-0180354 A1, and in PCT Application Pub. No. WO 2004/022709 A2.

IMMUNE EPITOPE - In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - In a preferred embodiment, a target cell is a cell associated with a pathogenic condition that can be acted upon by the components of the immune system, for example, a cell infected with a virus or other intracellular parasite, or a neoplastic cell. In another embodiment, a target cell is a cell to be targeted by vaccines and methods disclosed herein. Examples of target cells according to this definition include but are not necessarily limited to: a neoplastic cell and a cell harboring an intracellular parasite, such as, for example, a virus, a bacterium, or a protozoan. Target cells can also include cells that are targeted by CTL as a part of an assay to determine or confirm proper epitope liberation and processing by a cell expressing immunoproteasome, to determine T cell specificity or immunogenicity for a desired epitope. Such cells can be transformed to express the liberation sequence, or the cells can simply be pulsed with peptide/epitope.

TARGET-ASSOCIATED ANTIGEN (TAA) - a protein or polypeptide present in a target cell.

TUMOR-ASSOCIATED ANTIGEN (TuAA) - a TAA, wherein the target cell is a neoplastic cell. In alternate embodiments, a TuAA is an antigen associated with non-cancerous cells of the tumor such as tumor neovasculature or other stromal cells within the tumor microenvironment.

HLA EPITOPE - a polypeptide having a known or predicted binding affinity for a human class I or class II HLA complex molecule.

ANTIBODY - a natural immunoglobulin (Ig), poly- or monoclonal, or any molecule composed in whole or in part of an Ig binding domain, whether derived biochemically, or by use of recombinant DNA, or by any other means. Examples include *inter alia,* F(ab), single chain Fv, and Ig variable region-phage coat protein fusions.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or minor differences in length or composition of any non-coding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus may not be within the substantial similarity definition). Pairs of antibodies, or TCRs, that recognize the same epitope can be functionally similar to each other despite whatever structural differences exist. Testing for functional similarity of immunogenicity can be conducted by immunizing with the "altered" antigen and testing the ability of an elicited response, including but not limited to an antibody response, a CTL response, cytokine production, and the like, to recognize the target antigen. Accordingly, two sequences may be designed to differ in certain respects while retaining the same function.. Such designed sequence variants of disclosed or claimed sequences are among the embodiments of the present invention.

EXPRESSION CASSETTE - a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE - in some embodiments, an embedded epitope is an epitope that is wholly contained within a longer polypeptide; in other embodiments, the term also can include an epitope in which only the N-terminus or the C-terminus is embedded such that the epitope is not wholly in an interior position with respect to the longer polypeptide.

MATURE EPITOPE - a peptide with no additional sequence beyond that present when the epitope is bound in the MHC peptide-binding cleft.

EPITOPE CLUSTER - a polypeptide, or a nucleic acid sequence encoding it, that is a segment of a protein sequence, including a native protein sequence, comprising two or more known or predicted epitopes with binding affinity for a shared MHC restriction element. In preferred embodiments, the density of epitopes within the cluster is greater than the density of all known or predicted epitopes with binding affinity for the shared MHC restriction element within the complete protein sequence.

LIBERATION SEQUENCE - a designed or engineered sequence comprising or encoding a housekeeping epitope embedded in a larger sequence that provides a context allowing the housekeeping epitope to be liberated by processing activities including, for example, immunoproteasome activity, N terminal trimming, and/or other processes or activities, alone or in any combination.

CTLp - CTL precursors are T cells that can be induced to exhibit cytolytic activity. Secondary *in vitro* lytic activity, by which CTLp are generally observed, can arise from any combination of naive, effector, and memory CTL *in vivo.*

MEMORY T CELL - A T cell, regardless of its location in the body, that has been previously activated by antigen, but is in a quiescent physiologic state requiring re-exposure to antigen in order to gain effector function. Phenotypically they are generally CD62L⁻ CD44^{hi} CD107α⁻ IFN-γ⁻ LTβ⁻ TNF-α⁻ and is in G0 of the cell cycle.

EFFECTOR T CELL - A T cell that, upon encountering antigen, readily exhibits effector function. Effector T cells are generally capable of exiting the lymphatic system and entering the immunological periphery. Phenotypically they are generally CD62L⁻ CD44^{hi} CD107α+ IFN-γ+ LTβ⁺ TNF-α⁺ and actively cycling.

EFFECTOR FUNCTION - Generally, T cell activation generally, including acquisition of cytolytic activity and/or cytokine secretion.

INDUCING a T cell response - Includes in many embodiments the process of generating a T cell response from naive, or in some contexts, quiescent cells; activating T cells.

AMPLIFYING a T cell response - Includes in many embodiments, the process of increasing the number of cells, the number of activated cells, the level of activity, rate of proliferation, or similar parameter of T cells involved in a specific response.

ENTRAINMENT - Includes in many embodiments an induction that confers particular stability on the immune profile of the induced lineage of T cells. In various embodiments, the term "entrain" can correspond to "induce," and/or "initiate."

TOLL-LIKE RECEPTOR (TLR) - Toll-like receptors (TLRs) are a family of pattern recognition receptors that are activated by specific components of microbes and certain host molecules. As part of the innate immune system, they contribute to the first line of defense against many pathogens, but also play a role in adaptive immunity.

TOLL-LIKE RECEPTOR (TLR) LIGAND - Any molecule capable of binding and activating a toll-like receptor. Examples include, without limitation: poly IC A synthetic, double-stranded RNA known for inducing interferon. The polymer is made of one strand each of polyinosinic acid and polycytidylic acid, double-stranded RNA, unmethylated CpG oligodeoxyribonucleotide or other immunostimulatory sequences (ISSs), lipopolysacharide (LPS), β-glucans, and imidazoquinolines, as well as derivatives and analogues thereof.

IMMUNOPOTENTIATING ADJUVANTS - Adjuvants that activate pAPC or T cells including, for example: TLR ligands, endocytic-Pattern Recognition Receptor (PRR) ligands, quillaja saponins, tucaresol, cytokines, and the like. Some preferred adjuvants are disclosed in Marciani, D.J. Drug Discovery Today 8:934-943, 2003.

IMMUNOSTIMULATORY SEQUENCE (ISS) - Generally an oligodeoxyribonucleotide containing an unmethlylated CpG sequence. The CpG may also be embedded in bacterially produced DNA, particularly plasmids. Further embodiments include various analogues; among preferred embodiments are molecules with one or more phosphorothioate bonds or non-physiologic bases.

VACCINE - In preferred embodiments a vaccine can be an immunogenic composition providing or aiding in prevention of disease. In other embodiments, a vaccine is a composition that can provide or aid in a cure of a disease. In others, a vaccine composition can provide or aid in amelioration of a disease. Further embodiments of a vaccine immunogenic composition can be used as therapeutic and/or prophylactic agents.

IMMUNIZATION - a process to induce partial or complete protection against a disease. Alternatively, a process to induce or amplify an immune system response to an antigen. In the second definition it can connote a protective immune response, particularly proinflammatory or active immunity, but can also include a regulatory response. Thus in some embodiments immunization is distinguished from tolerization (a process by which the immune system avoids producing proinflammatory or active immunity) while in other embodiments this term includes tolerization.

ENCODE - an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic acid construct.

COVERAGE - the fraction or proportion of tumor cells expressing a particular TuAA or at least one TuAA from a set of selected TuAAs.

REDUNDANCY - the degree to which a population of tumor cells, or some subset of them, express more than one of a selected set of TuAAs.

CO-TARGETING - in preferred embodiments, co-targeting involves inducing and/or amplifying an immune response against a target cell, while also inducing an immune response against at least one other agent in the vicinity and/or *milieu* of a tumor. In some embodiments, agents within the vicinity and/or *milieu* of the tumor include, but are not limited to, cancer cells, stromal cells, including those associated with neovasculature, endothelial cells, fibroblasts, inflammatory cells, epithelial cells, autocrine factors, and paracrine factors. In some embodiments, tumor cells and stromal cells are specifically targeted. In other embodiments, an immune response is induced and/or amplified against neovasculature and other non-transformed, non-lymphoid cells within the tumor microenvironment. In still other embodiments, an immune response is induced against cancer cells and autocrine and/or paracrine factors produced by cells in the tumor microenvironment.

### Tumor Associated Antigens

Examples of TuAAs useful in embodiments disclosed herein include tyrosinase (SEQ. ID NO. 1), melan-A, (SEQ. ID NO. 2), SSX-2, (SEQ. ID NO. 3), PSMA (prostate-specific membrane antigen) (SEQ. ID NO. 4), MAGE-1, (SEQ. ID NO. 5), MAGE-3 (SEQ. ID NO. 6), NY-ESO-1, (SEQ. ID NO. 7), PRAME, (SEQ. ID NO.8), Her2/Neu (SEQ. ID NO. 9), mesothelin (SEQ. ID NOs. 10 and 11), VEGF-A (SEQ. ID NO. 12), and PLK1 (SEQ. ID NO. 13). The natural coding sequences for these proteins, or any segments within them, can be determined from their cDNA or complete coding (cds) sequences, SEQ. ID NOS. 14-26, respectively. The protein and cDNA sequences are identified by accession number and provided in the sequence listing filed herewith.

**Table 1. SEQ. ID NOS.**

| **SEQ. ID NO.** | **IDENTITY** | **ACCESSION NUMBER**** |
|---|---|---|
| 1 | Tyrosinase protein | P14679 |
| 2 | Melan-A protein | Q16655 |
| 3 | SSX-2 protein | NP_003138 |
| 4 | PSMA protein | NP_004467 |
| 5 | MAGE-1 protein | P43355 |
| 6 | MAGE-3 protein | P43357 |
| 7 | NY-ESO-1 protein | P78358 |
| 8 | PRAME protein | NP_006106 |
| 9 | Her2/Neu protein | P04626 |
| 10 | Mesothelin, isoform 1, protein | NP005814 |
| 11 | Mesothelin, isoform 2, protein | NP037536 |
| 12 | VEGF-A protein | P15692 |
| 13 | PLK1 protein | P53350 |
| 14 | Tyrosinase cDNA | NM_000372 |
| 15 | Melan-A cDNA | U06452 |
| 16 | SSX-2 cDNA | NM_003147 |
| 17 | PSMA cDNA | NM_004476 |
| 18 | MAGE-1 cds | M77481 |
| 19 | MAGE-3 cds | U03735 |
| 20 | NY-ESO-1 cDNA | U87459 |
| 21 | PRAME cDNA | NM_006115 |
| 22 | Her2/Neu cDNA | M11730 |
| 23 | Mesothelin, isoform 1, cDNA | NM005823 |
| 24 | Mestohelin, isoform 2, cDNA | NM013404 |
| 25 | VEGF-cDNA | NM_001025366 |
| 26 | Plk1 cDNA | NM_005030 |

| | | |
|---|---|---|
| **All accession numbers used here and throughout can be accessed through the NCBI databases, for example, through the Entrez seek and retrieval system on the world wide web. | | |

Tyrosinase is a melanin biosynthetic enzyme that is considered one of the most specific markers of melanocytic differentiation. Tyrosinase is expressed in few cell types, primarily in melanocytes, and high levels are often found in melanomas. The usefulness of tyrosinase as a TuAA is taught in U.S. Patent No. 5,747,271.

GP100, also known as PMell7, is another melanin biosynthetic protein expressed at high levels in melanomas. GP100 as a TuAA is disclosed in U.S. Patent No. 5,844,075.

Melan-A, also known as MART-1 (Melanoma Antigen Recognized by T cells), is another melanin biosynthetic protein expressed at high levels in melanomas. The usefulness of Melan-A/MART-1 as a TuAA is taught in U.S. Patent Nos. 5,874,560 and 5,994,523, as well as U.S. Patent No. 5,620,886.

SSX-2, also known as Hom-Mel-40, is a member of a family of highly conserved cancer-testis (CT) antigens (Gure, A.O. et al., Int. J. Cancer 72:965-971, 1997). Its identification as a TuAA is taught in U.S. Patent No. 6,025,191. Cancer-testis antigens are found in a variety of tumors, but are generally absent from normal adult tissues except testis. Expression of different members of the SSX family has been found in various tumor cell lines. Due to the high degree of sequence identity among SSX family members, similar epitopes from more than one member of the family will be generated and able to bind to an MHC molecule, so that some vaccines directed against one member of this family can cross-react and be effective against other members of this family.

MAGE-1 (melanoma-associated antigen-1), MAGE-2 (melanoma-associated antigen-2), and MAGE-3 (melanoma-associated antigen-3) are members of another family of cancer-testis antigens originally discovered in melanoma but found in a variety of tumors. The identification of MAGE proteins as TuAAs is taught in U.S. Patent No. 5,342,774 and in numerous subsequent patents. Currently there are 17 entries for (human) MAGE in the SWISS Protein database. There is extensive similarity among these proteins, such that in many cases, an epitope from one can induce a cross-reactive response to other members of the family. A few members of the MAGE family have not been observed in tumors, most notably MAGE-H1 and MAGE-D1, which are expressed in testes and brain, and bone marrow stromal cells, respectively. The possibility of cross-reactivity on normal tissue is ameliorated by the fact that they are among the least similar to the other MAGE proteins.

GAGE-1 is a member of the GAGE family of cancer testis antigens (Van den Eynde, B., et al., J. Exp. Med. 182: 689-698, 1995; U.S Patent Nos. 5,610,013; 5648226; 5,858,689; 6,013,481; and 6,069,001). The PubGene database currently lists 12 distinct accessible members, some of which are synonymously known as PAGE or XAGE. GAGE-1 through GAGE-8 have a very high degree of sequence identity, so most epitopes can be shared among multiple members of the family.

BAGE is a cancer-testis antigen commonly expressed in melanoma, particularly metastatic melanoma, as well as in carcinomas of the lung, breast, bladder, and squamous cells of the head and neck. Its usefulness as a TuAA is taught in U.S. Patent Nos. 5,683,88 and 5,571,711.

NY-ESO-1, also known as CTAG-1 (Cancer-Testis Antigen-1) and CAG-3 (Cancer Antigen-3), is a cancer-testis antigen found in a wide variety of tumors. NY-ESO-1 as a TuAA is disclosed in U.S. Patent 5,804,381. A paralogous locus encoding antigens with extensive sequence identity, LAGE-la/s and LAGE-lb/L, has been disclosed in publicly available assemblies of the human genome, and has been concluded to arise through alternate splicing. Additionally, CT-2 (or CTAG-2, Cancer-Testis Antigen-2) appears to be either an allele, a mutant, or a sequencing discrepancy of LAGE-lb/L. Due to the extensive sequence identity, many epitopes from NY-ESO-1 can also induce immunity to tumors expressing these other antigens. NY-ESO-1 and LAGE are virtually identical through amino acid 70. From amino acid 71 through 134 the longest run of identity between the two proteins is 6 residues, but potentially cross-reactive sequences are present. From amino acid 135 through 180, NY-ESO and LAGE-la/s are identical except for a single residue, but LAGE-lb/L is unrelated due to the alternate splice. The CAMEL and LAGE-2 antigens appear to derive from the LAGE-1 mRNA, but from alternate reading frames, thus giving rise to unrelated protein sequences. More recently, GenBank Accession AF277315.5, Homo sapiens chromosome X clone RP5-865E18, RP5-1087L19, complete sequence, reports three independent loci in this region which are labeled as LAGE1 (corresponding to CTAG-2 in the genome assemblies), LAGE2-A and LAGE2-B (both corresponding to CTAG-1 in the genome assemblies).

PRAME, also known as MAPE, DAGE, and OIP4, was originally observed as a melanoma antigen. Subsequently, it has been recognized as a cancer-testis (CT) antigen, but unlike many CT antigens, such as, MAGE, GAGE and BAGE, PRAME is expressed in acute myeloid leukemias. PRAME is a member of the MAPE family, which consists largely of hypothetical proteins with which it shares limited sequence similarity. The usefulness of PRAME as a TuAA is taught in U.S. Patent No. 5,830,753.

PSMA (prostate-specific membranes antigen), a TuAA described in U.S. Patent No. 5,538,866, is expressed by normal prostate epithelium and, at a higher level, in prostatic cancer. It has also been found in the neovasculature of non-prostatic tumors. PSMA can thus form the basis for vaccines directed to both prostate cancer and to the neovasculature of other rumors. This later concept is more fully described in U.S. Patent Application Pub. No. 2003-0046714 A1 and Pub. No. 2005-0260234 A1. Briefly, as tumors grow they recruit ingrowth of new blood vessels. This is understood to be necessary to sustain growth as the centers of unvascularized tumors are generally necrotic and angiogenesis inhibitors have been reported to cause tumor regression. Such new blood vessels, or neovasculature, express antigens not found in established vessels, and thus can be specifically targeted. By inducing CTL against neovascular antigens the vessels can be disrupted, interrupting the flow of nutrients to, and removal of wastes from, tumors, leading to regression.

Alternate splicing of the PSMA mRNA leads to a protein with an apparent start at Met₅₈, thereby deleting the putative membrane anchor region of PSMA as described in U.S. Patent No. 5,935,818. A protein termed PSMA-like protein, Genbank accession number AF261715, is nearly identical to amino acids 309-750 of PSMA, but has a different expression profile. Thus, the most preferred epitopes are those with an N-terminus located from amino acid 58 to 308.

PSA (prostate specific antigen) is a peptidase of the kallikrein family and a differentiation antigen of the prostate. Expression in breast tissue has also been reported. Alternate names include gamma-seminoprotein, kallikrein 3, seminogelase, seminin, and P-30 antigen. PSA has a high degree of sequence identity with the various alternate splicing products prostatic/glandular kallikrein-1 and -2, as well as kalikrein 4, which is also expressed in prostate and breast tissue. Other kallikreins generally share less sequence identity and have different expression profiles. Nonetheless, cross-reactivity that might be provoked by any particular epitope, along with the likelihood that that epitope would be liberated by processing in non-target tissues (most generally by the housekeeping proteasome), should be considered in designing a vaccine.

PSCA (prostate stem cell antigen) and also known as SCAH-2, is a differentiation antigen preferentially expressed in prostate epithelial cells, and overexpresssed in prostate cancers. Lower level expression is seen in some normal tissues including neuroendocrine cells of the digestive tract and collecting ducts of the kidney. PSCA is described in U.S. Patent No. 5,856,136.

Synaptonemal complex protein 1 (SCP-1), also known as HOM-TES-14, is a meiosis-associated protein and also a cancer-testis antigen (Tureci, O., et al., Proc. Natl. Acad. Sci. USA 95:5211-5216, 1998). As a cancer antigen its expression is not cell-cycle regulated and it is found frequently in gliomas, breast, renal cell, and ovarian carcinomas. It has some similarity to myosins, but with few enough identities that cross-reactive epitopes are not an immediate prospect.

The ED-B domain of fibronectin is also a potential target. Fibronectin is subject to developmentally regulated alternative splicing, with the ED-B domain being encoded by a single exon that is used primarily in oncofetal tissues (Matsuura, H. and S. Hakomori Proc. Natl. Acad Sci. USA 82:6517-6521, 1985; Carnemolla, B. et al., J. Cell Biol. 108:1139-1148, 1989; Loridon-Rosa, B. et al., Cancer Res. 50:1608-1612, 1990; Nicolo, G. et al., Cell Differ. Dev. 32:401-408, 1990; Borsi, L. et al., Exp. Cell Res. 199:98-105, 1992; Oyama, F. et al., Cancer Res. 53:2005-2011, 1993; Mandel, U. et al., APMIS 102:695-702, 1994; Farnoud, M.R. et al., Int. J. Cancer 61:27-34, 1995; Pujuguet, P. et al., Am. J. Pathol. 148:579-592, 1996; Gabler, U. et al., Heart 75:358-362, 1996; Chevalier, X. Br. J. Rheumatol. 35:407-415, 1996; Midulla, M. Cancer Res. 60:164-169, 2000).

The ED-B domain is also expressed in fibronectin of the neovasculature (Kaczmarek, J. et al., Int. J. Cancer 59:11-16, 1994; Castellani, P. et al., Int. J. Cancer 59:612-618, 1994; Neri, D. et al., Nat. Biotech. 15:1271-1275, 1997; Karelina, T.V. and A.Z. Eisen Cancer Detect. Prev. 22:438-444, 1998; Tarli, L. et al., Blood 94:192-198, 1999; Castellani, P. et al., Acta Neurochir (Wien) 142:277-282, 2000). As an oncofetal domain, the ED-B domain is commonly found in the fibronectin expressed by neoplastic cells in addition to being expressed by the neovasculature. Thus, CTL-inducing vaccines targeting the ED-B domain can exhibit two mechanisms of action: direct lysis of tumor cells, and disruption of the tumor's blood supply through destruction of the tumor-associated neovasculature. As CTL activity can decay rapidly after withdrawal of vaccine, interference with normal angiogenesis can be minimal. The design and testing of vaccines targeted to neovasculature is described in U.S. Patent Application Pub. No. 2003-0046714 A1 and Pub. No. 2005-0260234 A1.

Carcinoembryonic antigen (CEA) is a paradigmatic oncofetal protein first described in 1965 (Gold and Freedman, J. Exp. Med. 121: 439-462, 1965). Fuller references can be found in the Online Mendelian Inheritance in Man; record * 114890. It has officially been renamed carcinoembryonic antigen-related cell adhesion molecule 5 (CEACAM5). Its expression is most strongly associated with adenocarcinomas of the epithelial lining of the digestive tract and in fetal colon. CEA is a member of the immunoglobulin supergene family and the defining member of the CEA subfamily.

Survivin, also known as Baculoviral IAP Repeat-Containing Protein 5 (BIRC5), is another protein with an oncofetal pattern of expression. It is a member of the inhibitor of apoptosis protein (IAP) gene family. It is widely over-expressed in cancers (Ambrosini, G. et al., Nat. Med 3:917-921, 1997; Velculiscu V.E. et al., Nat. Genet. 23:387-388, 1999) and its function as an inhibitor of apoptosis is believed to contribute to the malignant phenotype.

HER2/NEU is an oncogene related to the epidermal growth factor receptor (van de Vijver, et al., New Eng. J. Med. 319:1239-1245, 1988), and apparently identical to the c-ERBB2 oncogene (Di Fiore, et al., Science 237: 178-182, 1987). The over-expression of ERBB2 has been implicated in the neoplastic transformation of prostate cancer. As with HER2, it is amplified and over-expressed in 25-30% of breast cancers among other tumors where expression level is correlated with the aggressiveness of the tumor *(*Slamon, et al., New Eng. J. Med. 344:783-792, 2001). A more detailed description is available in the Online Mendelian Inheritance in Man; record *164870.

MESOTHELIN is an antigen originally found in mesotheliomas but also known to be upregulated in many pancreatic and ovarian cancers. Its use as a vaccine target and useful epitopes are described in Thomas, A.M. et al., J. Exp. Med. 200:297-306, 2004.

Vascular Endothelial Growth Factor (VEGF-A or VEGF) is a mitogenic protein structurally related to platelet-derived growth factor, but with narrower mitogenic activity focused on vascular endothelial cells. The protein and its receptors are important to tumor growth and their potential as targets of cancer therapies has been noted (Folkman, J. Nature Med. 1: 27-31, 1995). A more detailed description is available in the Online Mendelian Inheritance in Man; record* 192240.

PLK1 is an intracellular serine/threonine kinase that plays a critical role in cell cycle regulation and in DNA damage responses. Mutation or knock down of PLK1 result in abnormal mitosis, cell cycle arrest and apoptosis (Reagan-Shaw S. and Ahmad N., FASEB J. 19:611, 2005). PLK1 belongs to the Polo-like kinase family of structurally conserved kinases. The PLK1 family contains two conserved regions, an N-terminal kinase domain and a C-terminal non-catalytic Polo box region (Lowery DM et al., Oncogene 24:248.2005) responsible for subcellular localization. PLK1 is predominantly found in the cytoplasm during interphase and localizes to the nucleus during mitosis (Takai N et al., Oncogene 24:2872005). Nuclear localization of PLK1 is essential for its biological function (Lee KS et al., Proc Natl Acad Sci USA 95:9301, 1998). While most classical tumor associated antigens are not known to participate in tumor initiation or disease progression, PLK1 has been shown to drive tumor growth. Thus, in some embodiments, PLK1 can be considered a tumor-associated antigen that can be used as a target for cancer immunotherapy. Based on its expression profile, PLK1 is expressed in proliferating cells, with elevated expression found in most solid tumors. This expression pattern renders PLK1 a valid target for active and passive immunotherapy.

Further examples of tumor-associated antigens include MelanA (MART-I), gp100 (Pmel 17), tyrosinase, TRP-1, TRP-2, MAGE-1, MAGE-3, BAGE, GAGE-1, GAGE-2, pl5(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, pl85erbB2, pl80erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like.

Additional tumor-associated antigens are described in Chen, YT, "Identification of human tumor antigens by serological expression cloning: an online review on SEREX" Cancer Immun. 2004 [updated 2004 Mar 10; cited 2004 Apr 1] *at* world wide web cancerimmunotherapy.org/SEREX/; and Renkvist, N. et al., "A listing of tumor antigens recognized by T cells," Cancer Immunology Immunotherapy, 50:3-15 (2001.

Table 2, adapted from Scanlan et al., "The cancer/testis genes: Review, standardization, and commentary," Cancer Immunity 4:1 (January 23, 2004), provides a listing of CT Antigens. Table 3 provides the frequency of mRNA expression in various tumor types for the CT antigens in Table 2. Scanlan et al., "The cancer/testis genes: Review, standardization, and commentary," Cancer Immunity 4:1 (January 23, 2004).

**Table 2**

| Listing of CT genes | | |
|---|---|---|
| **CT Identifier** | **Transcript/Transcript family** | **Family Members/CT Identifier (Synonyms)** |
| CT1 | MAGEA | MAGEA1/CT1.1, MAGEA2/CT1.2, MAGEA3/CT1.3, MAGEA4/CT1.4, MAGEA5/CT1.5, MAGEA6/CT1.6, MAGEA7/CT1.7, MAGEA8/CT1.8, MAGEA9/CT.9, MAGEA10/CT1.10, MAGEA11/CT1.11, MAGEA12/CT1.12 |
| CT2 | BAGE | BAGE/CT2.1, BAGE2/CT2.2, BAGE3/CT2.3, BAGE4/CT2.4, BAGE5/CT2.5 |
| CT3 | MAGEB | MAGEB1/CT3.1, MAGEB2/CT3.2, MAGEB5/CT3.3, MAGEB6/CT3.4 |
| CT4 | GAGE1 | GAGE1/CT4.1, GAGE2/CT4.2, GAGE3/CT4.3, GAGE4/CT4.4, GAGE5/CT4.5, GAGE6/CT4.6, GAGE7/CT4.7, GAGE8/CT4.8 |
| CT5 | SSX | SSX1/CT5.1, SSX2/CT5.2a, SSX2/CT5.2b, SSX3/CT5.3, SSX4/CT5.4 |
| CT6 | NY-ESO-1 | NY-ESO-1/CT6.1, LAGE-la/CT6.2a, LAGE-1b/CT6.2b |
| CT7 | MAGEC1 | MAGEC1/CT7.1, MAGEC3/CT7.2 |
| CT8 | SYCP1 | SYCP1/CT8 |
| CT9 | BRDT | BRDT/CT9 |
| CT10 | MAGEE1 | MAGEE1/CT10 |
| CT11 | CTp11/SPANX | SPANXA1/CT11.1, SPANXB1/CT11.2, SPANXC/CT11.3, SPANXD/CT11.4 |
| CT12 | XAGE-1/GAGED | XAGE-1a/CT12.1a, XAGE-1b/CT12.1b, XAGE-1c/CT12.1c, XAGE-1d/CT12.1d, XAGE-2/CT12.2, XAGE-3a/CT12.3a, XAGE-3b/CT12.3b, XAGE-4/CT12.4 |
| CT13 | HAGE | HAGE/CT13 |
| CT14 | SAGE | SAGE/CT14 |
| CT15 | ADAM2 | ADAM2/CT15 |
| CT16 | PAGE-5 | PAGE-5/CT16.1, CT16.2 |
| CT17 | LIP1 | LIP1/CT17 |
| CT18 | NA88 | NA88/CT12 |
| CT19 | IL13RA1 | IL13RA1/CT19 |
| CT20 | TSP50 | TSP50/CT20 |
| CT21 | CTAGE-1 | CTAGE-1/CT21.1, CTAGE-2/CT21.2 |
| CT22 | SPA 17 | SPA17/CT22 |
| CT23 | OY-TES-1 | OY-TES-1/CT23 |
| CT24 | CSAGE | CSAGE/CT24.1, TRAG3/CT24.2 |
| CT25 | MMA1/DSCR8 | MMA-1a/CT25.1a, MMA-1b/CT25.1b |
| CT26 | CAGE | CAGE/CT26 |
| CT27 | BORIS | BORIS/CT27 |
| CT28 | HOM-TES-85 | HOM-TES-85/CT28 |
| CT29 | AF15q14/D40 | D40/CT29 |
| CT30 | E2F-like/HCA661 | HCA661/CT30 |
| CT31 | PLU-1 | PLU-1/CT31 |
| CT32 | LDHC | LDHC/CT32 |
| CT33 | MORC | MORC/CT33 |
| CT34 | SGY-1 | SGY-1/CT34 |
| CT35 | SPO11 | SPO11/CT35 |
| CT36 | TPX1 | TPX-1/CT36 |
| CT37 | NY-SAR-35 | NY-SAR-35/CT37 |
| CT38 | FTHL17 | FTHL17/CT38 |
| CT39 | NXF2 | NXF2/CT39 |
| CT40 | TAF7L | TAF7L/CT40 |
| CT41 | TDRD1 | TDRD1/CT41.1, NY-CO-45/CT41.2 |
| CT42 | TEX15 | TEX15/CT42 |
| CT43 | FATE | FATE/CT43 |
| CT44 | TPTE | TPTE/CT44 |
| --- | PRAME | (MAPE, DAGE) |

**Table 3.**

| **CT Family (Member)** | **Frequency (%) of Expression in Tumor Type** | | | | | | | | | | | | | | | | **Ref** |
|---|---|---|---|---|---|---|---|---|---|---|---|---|---|---|---|---|---|
| | **Blad** | **Brn** | **Brst** | **Col** | **Eso** | **Gas** | **H/N** | **Liver** | **Leuk/Lymph** | **Lung (NSCLC)** | **Mel** | **Ov** | **Pancr** | **Pros** | **Renal** | **Sarc** | |
| MAGEA1/CT1.1 | 22 | - | 18 | 2 | 53 | 29 | 28 | 80 | 0 | 49 | 48 | 28 | - | 15 | 0 | 14 | 44 |
| BAGE1/CT2.1 | 15 | - | 10 | 0 | - | - | 8 | - | 0 | 4 | 26 | 15 | - | 0 | 0 | 6 | 44 |
| MAGEB1/CT3.1 | 0 | 0 | 17 | 0 | - | 0 | 0 | - | 0 | 14 | 22 | - | - | 0 | 0 | 9 | 45 |
| GAGE/CT4.1 | 12 | - | 9 | 0 | - | - | 19 | 38^{b} | 1 | 19 | 28 | 31 | - | 10 | 0 | 25 | 44 |
| SSX2/CT5.2 | 44 | 6 | 7 | 12 | - | - | 35 | 9^{b} | 36 | 16 | 35 | | - | 40 | 5 | 50 | 46 |
| NY-ESO-1/CT6.1 | 80 | 0 | 30 | 0 | - | 0 | | 29 | 0 | 17 | 34 | 25 | 0 | 25 | 9 | 0 | 8 |
| MAGEC1/CT7.1 | 44 | - | 30 | 10 | - | - | 36 | - | - | 33 | 70 | - | - | - | - | 60 | 20 |
| SYCP1/CT8 | - | 47 | 20 | 0 | - | 7 | - | 28^{b} | 0 | 7 | 14 | 0 | - | 0 | 8 | 0 | 9 |
| BRDT/CT9 | 0 | - | 0 | 0 | 8 | - | 8 | - | - | 25 | 0 | - | - | - | 0 | - | 16 |
| MAGEE1/CT10 | 44 | - | 38 | 0 | - | - | 36 | - | - | 24 | 50 | - | - | - | - | 0 | 12 |
| SPANXC/CT11.3 | 9 | - | 25 | 22 | 0 | - | - | - | - | 33 | 70 | - | 0 | - | - | - | 14 |
| XAGE-1a/CT12.1a | - | - | - | - | - | - | - | - | - | - | 8 | | - | - | - | 22 | 47 |
| HAGE/CT13 | 24 | 37 | 5 | 31 | 27 | - | - | 20 | 9 | 32 | 17 | - | - | 22 | 6 | 20 | 13 |
| SAGE/CT14 | 12 | 0 | 5 | 0 | 20 | - | 17 | - | 4 | 22 | 4 | - | - | 0 | 5 | 5 | 13 |
| ADAM2/CT15 | - | - | 0 | 0 | - | - | - | - | - | 0 | 0 | 0 | - | - | 12 | - | 17 |
| PAGE-5/CT16 | - | - | 5 | 11 | - | - | - | - | - | 39 | 22 | 0 | - | - | 44 | - | 17 |
| LIP1/CT17 | - | | 5 | 0 | - | - | - | - | - | 0 | 0 | 0 | - | - | 25 | - | 17 |
| NA88/CT18 | - | - | - | - | - | - | - | - | - | - | 11 | - | - | - | - | - | 48 |
| TSP50/CT20 | - | - | 28 | - | - | - | - | - | - | - | - | | | - | - | - | 49 |
| CTAGE-1/CT21.1 | - | - | - | - | - | - | - | - | 35 | - | - | - | - | - | - | - | 50 |
| SPA17/CT22 | - | - | - | - | - | - | - | - | 26 | - | - | - | - | - | - | - | 51 |
| OYTES1/CT23 | 28 | - | 40 | 15 | - | 0 | - | 40 | - | 20 | - | - | - | - | 0 | - | 52 |
| MMA1a/CT25.1a | - | - | 0 | 0 | 0 | - | - | - | - | 40 | 26 | - | 0 | - | - | 18 | 15 |
| CAGE/CT26 | - | - | - | - | - | 89 | - | - | - | 100 | - | - | - | - | - | - | 53 |
| HOMTES85/CT28 | - | 35 | 0 | 10 | - | - | - | 19 | - | 28 | 36 | 32 | - | 0 | - | - | 54 |
| D40/CT29 | - | 20 | - | 13 | - | 0 | - | - | - | 41 | - | 36 | 27 | - | - | - | 55 |
| HCA661/CT30 | 0 | - | - | - | - | 0 | 0 | 29 | - | - | 20 | | - | - | - | - | 56 |
| PLU-1/CT31 | - | - | 86 | - | - | - | - | - | - | - | - | - | - | - | - | - | 27 |
| LDHC/CT32 | - | - | 35 | 15 | - | - | - | - | - | 47 | 44 | 42 | - | 37 | 57 | - | 18 |
| MORC/CT33 | - | - | 0 | 0 | - | - | - | - | - | 18 | 18 | 14 | - | 0 | 0 | - | 18 |
| SGY-1/CT34 | - | - | 20 | 0 | - | - | - | | - | 12 | 25 | 57 | - | 12 | 0 | - | 18 |
| SP011/CT35 | - | - | 0 | 0 | - | - | - | - | - | 0 | 6 | 0 | - | 0 | 0 | - | 18 |
| TPX1/CT36 | - | - | 15 | 0 | - | - | - | - | - | - | 6 | 14 | - | 37 | 14 | - | 18 |
| NYSAR35/CT37 | 42 | - | 23 | 0 | 8 | - | - | - | - | 17 | 6 | 8 | - | - | 0 | 8 | 57 |
| FTHL17/CT38 | 22 | - | 14 | 0 | 0 | | 10 | - | 0 | 25 | 0 | - | - | 0 | 0 | 0 | 58 |
| NXF2/CT39 | 19 | - | 0 | 11 | 12 | - | 5 | - | 0 | 15 | 55 | - | - | 14 | 0 | 27 | 58 |
| TAF7L/CT40 | 10 | - | 0 | 0 | 0 | - | 10 | - | 0 | 9 | 21 | - | - | 0 | 0 | 12 | 58 |
| TDRD1/CT41.1 | 28 | - | 37 | 0 | 10 | - | 22 | - | 5 | 5 | 0 | - | - | 38 | 0 | 0 | 58 |
| TEX15/CT42 | 21 | - | 0 | 0 | 20 | - | 11 | - | 0 | 21 | 27 | - | - | 12 | 33 | 28 | 58 |
| FATE/CT43 | - | - | - | 21 | - | 7 | - | 66 | - | 0 | - | - | - | - | - | - | 19 |
| TPTE/CT44 | - | - | - | 0 | - | 0 | - | 39 | - | 36 | - | - | - | - | - | - | 19 |

| | | | | | | | | | | | | | | | | | |
|---|---|---|---|---|---|---|---|---|---|---|---|---|---|---|---|---|---|
| ^{a}Abbreviations: Blad, bladder; Brn, brain; Brst, breast; Col, colon; Gas, gastric; H/N, head and neck; Leuk, leukemia; Lymph, lymphoma, NSCLC, non-small cell lung carcinoma; Mel, melanoma; Ov, ovarian; Pancr, pancreatic; Pros, prostate; Sarc, sarcoma; Ref, reference. ^{b}Reference 59. | | | | | | | | | | | | | | | | | |

Many antigens listed in the tables above have no documented role in maintaining the transformed phenotype of a cell, and thus, a transformed cell may lose expression of such antigens without affecting the viability of the cell or the malignancy of the disease. Although some CT antigens, such as the SSX proteins, are known to be transcriptional regulators, their role, if any, in tumorigenicity remains obscure. It has also recently emerged that PRAME can repress signaling through the retinoic acid receptor to inhibit retinoic acid-induced differentiation, growth arrest, and apoptosis, thereby suggesting that PRAME over-expression can impart tumor cells with a growth or survival advantage. It would be advantageous to target an antigen, the loss of which necessarily would affect viability of the cell or the malignancy of the disease. Such antigens are included in preferred embodiments of the combinations of tumor and/or tumor and stromal antigens disclosed herein.

However, many genes involved in the regulation of proliferation are important in oncogenesis primarily in mutated form so that specific immune attack on the mutated form depends on the mutation being appropriately placed within an epitope. Furthermore, epitopes present in the wild type molecule (whether or not the targeted molecule is wild type or mutant) will generally only be considered if there is a substantial difference in expression level between transformed and normal cells, or at least the normal cells of vital organs. Thus appropriate antigens have been difficult to recognize.

The expression of PLK1 is regulated during cell cycle progression as well as throughout the various disease stages. PLK1 expression is minimal during the initial phases of cell division, begins to increase during G2 and peaks at M phase. PLK1 is targeted for degradation by the proteasome pathway after cells exit from mitosis. In normal tissues, PLK1 is expressed in adult organs containing highly proliferative cells, such as in the spleen, placenta, ovary, and testis. Its expression is undetected in vital organs such as heart, lung, liver, brain, intestines, smooth muscle, and skin. Wild type PLK1 is overexpressed in tumor tissues, including breast, prostate, ovarian, non-small cell lung, head/neck, colon, pancreatic, endometrial, and esophageal carcinomas (Table 4). Importantly, the level of PLK1 expression often correlates with more advanced stages of tumor progression and poor prognosis (Wolf G et al., Oncogene 14:543, 1997; Takai N et al., Cancer Lett. 164:41, 2001). This has been specifically demonstrated in the case of NSCLC, esophageal, and head/neck carcinomas. Thus, PLK1 is a viable target for cancer immunotherapy for those tumors in which it is overexpressed.

**Table 4. Plk-1 Expression Profile**

| **Tumor** | **Penetrance** | **Expression Level** | **Reference** |
|---|---|---|---|
| Lung (NSCLC) | >90% | Med/strong | Wolf G et al, Oncogene 14:543, 1997. |
| Ovarian | >85% | Low/strong | Takai N et al, Cancer Lett. 164:41, 2001 |
| Breast | 43 % | Med/strong | Weichert W et al, Virchows Arch, 446: 442, 2005. |
| Prostate | 53 % | Med/strong | Weichert Wet al, Curr. Biol. 101:4419, 2004. |
| Colorectal | 73% | Med/strong | Takahashi T et al, Cancer Sci. 94:148 2003. |
| Pancreatic | 48% | Med/strong | Gray JP et al, Mol Cancer Ther. 3:641,2004. |
| Head & Neck | 72% | Med/strong | Knecht R et al, Cancer Res. 59:2794, 1999. |
| Melanoma | 53 % | Med | Strebhardt K et al, JAMA. 283:479, 2000. |
| Esophageal | 96% | Med | Tokumitsu Y et al, Int J Oncol. 15:687, 1999. |

Additional antigens associated with tumor neovasculature are VEGFR2 (vascular endothelial growth factor receptor 2) described in U.S. Patent No. 6,342,221 and Tie-2, an endothelium specific receptor tyrosine kinase, which is described in WO 99/43801.

In addition to disrupting blood flow to tumors that can be achieved using anti-neovasculature agents such as those recited above, co-targeting molecules expressed on cancer cells as well as molecules expressed on underlying non-transformed stromal cells (including neovasculature as well as interstitial tissue, for example) can also improve the effectiveness of the disclosed methods and compositions in limiting tumor growth and promoting cancer regression by other mechanisms. Stroma encompasses neovasculature as well as fibroblasts, and in general, all non-transformed, non-lymphoid cells within a tumor microenvironment. For example, immune mediated attack of the endothelial cells (via cytotoxic T lymphocytes (CTLs) or antibody dependent cytotoxic cells (ADCC) can result in neovasculature permeabilization and initiation of inflammatory events that result in recruitment and translocation of immune effectors, such as CTLs, targeting the neoplastic cells within primary tumor and metastatic lesions. Moreover, as attacks based, for example, on T cell recognition of endothelial cell MHC-peptide complexes occur in the luminal environment, any immune suppressive influence of the tumoral environment is minimized. Compared to strategies targeting only cancer cells, methods to co-target associated stromal tissue thus improve the efficacy of the former. In some embodiments, the efficacy is synergistically enhanced. Similarly, compared to strategies targeting neovasculature only, methods to co-target cancer cells improve the overall therapeutic effect by attacking lesions, including those of limited size and vascularization, especially those adversely located within vital organs. With regard to neovasculature, co-targeting VEGFRs (such as II), CD55 and PSMA as well as other molecules expressed by neovasculature, can be accomplished by generating CTL or antibodies with capability to initiate ADCC or complement activated cell injury. Alternatively, initial endothelial injury can be brought about through passive immunotherapy using available anti-angiogenic antibodies.

In addition or alternatively, co-targeting target-associated antigens, together with growth, metastasis, or survival promoting factors produced by cancer cells or non-transformed cells that are found in the extracellular compartment (diffusing or associated with the extracellular matrix), can also result in a more substantial therapeutic effect. By co-targeting antigens expressed within or on cancer cells as well as factors that exert autocrine or paracrine effects (growth, survival, and/or invasiveness), the pathogenic process can be slowed or disrupted to a significant degree. Co-targeting autocrine or paracrine factors (such as, but not limited to, NF-κB activating molecules - CXCL1, CXCL8, CCL2; or growth factors such as, but not limited to, chorionic-gonadotropic hormone gastrin, and VEGF-A) can be carried out by co-induction of neutralizing antibodies or secondarily, by CTLs recognizing cells that produce such factors.

The interaction between transformed and stromal cells is mediated by VEGF-A. VEGF-A has been shown to play a key role in the establishment and functionality of tumor neovasculature; thus, deprival of VEGF-A by using specific passive immunotherapy (via anti-VEGF-A antibodies such as bevacizumab (AVASTIN®) resulted in control of tumor progression and metastatic disease for colorectal, lung, breast, ovarian carcinoma and other cancers. The mechanism of action likely involves direct neutralization of VEGF-A, thereby slowing down the establishment and progression of neovasculature. Neutralization of VEGF-A by passive immunotherapy can be combined with the active immunotherapies disclosed herein. VEGF-A can also be used as the target antigen for active or T-cell based immunotherapy directed against cells that produce VEGF-A in excess (most likely a subpopulation of tumoral cells, and most often the transformed cancer cells within the tumor environment). Such a strategy can be more effective in controlling a tumoral process both by mediating immune damage of cancer cells that express VEGF-A and by depriving the tumor neovasculature of an essential growth factor.

Overall, co-targeting multiple elements of biological importance for tumor growth and metastasis can limit progression of the malignant process by impacting the processes of clonal selection, immune evasion and escape. Thus, co-targeting stroma-associated antigens provides an additional mode of attack in that such activities are inhibited and/or disrupted.

One of skill in the art will appreciate that any other antigen or protein associated with vascular or other tumor-associated stromal cells can be a target for the immunogenic compositions, including those that are presently known and those yet to be identified.

### Compositions

Immunogenic compositions, including, for example, vaccines, can be prepared using whole antigen or an epitopic peptide. Peptide immunogens can be readily prepared using standard peptide synthesis means known in the art, for example. Immunogens can be prepared commercially by one of numerous companies that do chemical synthesis. An example of such a company is American Peptides, Inc., where the distributor is CLINALFA AG (Laufelfingen, Switzerland). The antigens or immunogens can be prepared in accordance with GMP standards and purity can be assessed by analytical HPLC. The product can be characterized by amino-acid analysis and tested for sterility and the absence of pyrogens.

The immunogenic compositions can also include adjuvants or other biological response modifiers (BRMs).

An antigen can be delivered to an animal's system either directly or indirectly. For example, a polypeptide can be delivered directly as the polypeptide, or it can be delivered indirectly, for example, using a DNA construct or vector, or a recombinant virus that codes for the desired antigen. Any vector driving expression in a professional antigen presenting cell can be suitable for this purpose. In indirect delivery, the antigen is expressed in the cell, then presented by the MHC Class I on the surface of the cell to stimulate a CTL response. Expression of a secreted form of the antigen can be useful to induce an antibody response recognizing antigens that are membrane proteins.

In a preferred embodiment, an encoded antigen can be delivered in the form of a naked plasmid expression vector. Particularly useful constructs are disclosed in U.S. Patent Application Pub No. 2003-0138808; and PCT Application Pub. No. WO 2004/018666; U.S. Patent Application Pub. No. 2003-0228634 A1, Pub. No. 2004-0132088 A1 and Pub. No. 2004-0203051 A1; U.S. Patent No. 6,709,844, and U.S. Patent Application Pub. No. 2003-0180949 A1 and in U.S. Patent Application Pub. No. 2003-0215425 A1, Pub. No. 2005-0130920 A1, Pub. No. 2007-0184062 A1. Additional methodology, compositions, peptides, and peptide analogues are disclosed in U.S. Patent Application Pub. No. 2006-0063913 A1 and U.S. Patent Application Pub. No. 2006-0094661 A1. The feasibility of and general procedures related to the use of naked DNA for immunization are described in U.S. Patent No. 5,589,466 and in U.S. Patent No. 5,679,647 The former teaches only intramuscular or intradermal injection while the latter teaches only administration to skin or mucosa.

In a preferred embodiment, the antigen can be administered directly to the lymphatic system. Intranodal administration for the generation of CTL is taught in U.S. Patent No. 6,994,851, and U.S. Patent No. 6,977,074, and in PCT Application Pub. No. WO 99/02183 A2. Single bolus injection intra lymph node (i.ln.) required only 0.1% of the dose required in order to obtain a similar level of CTL response by intramuscular (i.m.) injection. Therefore a protective response can be established against systemic viral infection with a single bolus delivered i.ln., but not with a dose nearing the practical limit delivered i.m. Repeated bolus injections i.m. failed to establish a protective response against a peripheral virus infection or transplanted tumor, whereas lower doses administered i.ln. were completely effective. Particularly useful intranodal immunization protocols are taught in U.S. Patent Application Pub. No. 2005-0079152 A1.

A class of epitopes that can be advantageous in anti-cancer immunogenic compositions are housekeeping epitopes. These are produced through the action of the housekeeping (or standard) proteasome. Housekeeping epitopes can be liberated from the translation product of expression vectors through proteolytic processing by the immunoproteasome of professional antigen presenting cells (pAPC). In one embodiment, sequences flanking the housekeeping epitope(s) can be altered to promote cleavage by the immunoproteasome at the desired location(s). Housekeeping epitopes, their uses, and identification are described in U.S. Patent Application Pub. No. 2003-0215425 A1, Pub No. 2005-0130920 A1, Pub. No. 2007-0184062 A1, and U.S. Patent No. 6,861,234 and U.S. Patent Application Pub. No. 2005-0069982 A1.

Examples of housekeeping epitopes are disclosed in U.S. Patent Application Publication No. 2003-0220239 A1; and Pub. No. 2004-0180354, and PCT Application Pub. No. WO 04/022709 A2.

In other embodiments, the housekeeping epitope(s) can be flanked by arbitrary sequences or by sequences incorporating residues known to be favored in immunoproteasome cleavage sites. As used herein the term "arbitrary sequences" refers to sequences chosen without reference to the native sequence context of the epitope, their ability to promote processing, or immunological function. In further embodiments multiple epitopes can be arrayed head-to-tail. These arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. Further, the arrays can be of any other similar arrangement. There is no restriction on placing a housekeeping epitope at the terminal positions of the array. The vectors can additionally contain authentic protein coding sequences or segments thereof containing epitope clusters as a source of immune epitopes. The term "authentic" refers to natural protein sequences.

Epitope clusters and their uses are described in U.S. Patent Application Pub. No. 2003-0215425 A1, Pub. No. 2005-0130920 A1 and Pub. No. 2007-0184062 A1.

In another embodiment an encoded antigen can be delivered in the form of a viral vector. A wide array of viruses with modified genomes adapted to express interposed reading frames but often no, or at least a reduced number of, viral proteins are known in the art, including without limitation, retroviruses including lentiviruses, adenoviruses, parvoviruses including adeno-associated virus, herpesviruses, and poxviruses including vaccinia virus. Such viral vectors facilitate delivery of the nucleic acid component into the cell allowing for expression. A subset of these vectors, such as retroviruses and parvoviruses, promote integration of their nucleic acid component into the host genome, whereas others do not.

Bacteria can also serve as vectors, that is, they can be used to deliver a nucleic acid molecule capable of causing expression of an antigen. For example, a strain of Listeria monocytogenes has been devised that effects its own lysis upon entering the cytosol of macrophages (its normal target), thereby releasing plasmid from which antigen is subsequently expressed (Dietrich, G. et al., Biotechnology 16:181-185, 1998). *Shigela flexneri* and *Escherichia coli* have been similarly used (Sizemore, D.R. et al., Science 270:299-302, 1995, and Courvalin, P. et al., Life Sci. 318:1207-1212, 1995, respectively).

The use of microbial vectors for nucleic acid delivery can be complicated by the immune reactions the vectors themselves provoke. When prolonged or repeated administration is required, antibody elicited by the earlier treatment can prevent useful quantities of the vector from ever reaching its intended host. However, by direct administration intra lymph node, for example, the combination of proximity to host cells and the much reduced effective dose makes it possible to administer a dose capable of evading or overwhelming an existing antibody titer.

The word vector has been used, here and elsewhere, in reference to several modalities and variously modified (e.g., expression vector, viral vector, delivery vector, etc.). The underlying principle is that a nucleic acid capable of causing expression of an antigen, rather than the antigen itself, ultimately arrives in an APC. Unless modified, explicitly or by local context, in preferred embodiments, the term vector as used herein is intended to encompass all such possibilities.

The techniques discussed above are distinct from the approach of modifying the microbial genome, including extra-chromosomal DNA, such that the antigen is produced as a component of the microbe, which is then itself administered as the immunogen. Examples of microbes used in the genomic modification approach include viruses, bacteria, fungi, and protozoa. In embodiments described herein, the compositions, including the vaccines, can include the already synthesized antigen or a nucleic acid capable of causing an APC to express the antigen *in vivo.* In alternative embodiments, combinations of these two techniques are used. For example, one embodiment contemplates the use of a virus vector as discussed above that also incorporates a target epitope into a capsid or envelope protein.

Antigens may be used alone or may be delivered in combination with other antigens or with other compounds such as cytokines. Cytokines that are known to enhance immune stimulation of CTL responses, include, for example, GM-CSF, IL- 12, IL-2, TNF, IFN, IL-18, IL-3, IL-4, IL-8, IL-9, IL-13, IL-10, IL-14, IL-15, G-SCF, IFN alpha, IFN beta, IFN gamma, TGF alpha, TGF beta, and the like. Cytokines are known in the art and are readily available in the literature or commercially. Many animal and human tumors have been shown to produce cytokines, such as IL-4, IL-10, TGF-B, that are potent modulators of the immune response and that protect tumors from immune-mediated destruction. The production of IL-4, IL-10 or TGF-B by tumors may achieve this protective effect by suppressing the induction of cellular immunity, including the elaboration of CTL responses. Alternatively, cytokines that support CTL responses can be exogenously added to help in the balance between induction of anti-tumor cell mediated and non-tumor-destructive humoral responses. Several such exogenous cytokines show utility in experimental mouse vaccination models which are known to enhance CTL responses, including GM-CSF, IFN and IL-2. An example of an effective exogenous cytokine that can be used is GM-CSF. GM-CSF is reported to enhance the expression of the so called "co-stimulatory" molecules, such as B7-1 or B7-2 on antigen presenting cells (APC). These co-stimulatory molecules are important players in the variety of interactions that occur during stimulation of CTL by APC. Moreover, GM-CSF is known to induce activation of APCs and to facilitate growth and differentiation of APCs, thereby making these APCs important CTL stimulating cells available both in greater numbers and potency.

Immunogenic compositions can additionally contain non-target antigens in order to improve the response to the target antigen. Thus, co-induction of a helper response, such as Th and/or B cell immunity against non-self or foreign antigens not expressed within the tumoral process or in the body, can result in a substantial improvement in the magnitude and quality of the immune response to the "self or "self-modified" target antigens expressed within the tumor or underlying stroma. For example, co-initiating a Th immune response against a non-target antigen such as tetanus toxoid can result in the generation of helper cells with bystander effect relative to generation of CTL or B cell responses against the target tumor or self antigens. Any defined sequence expressing or encompassing peptide motifs that bind to at least one class II MHC protein expressed by recipient, where such sequences are non-homologous or contain non-homologous segments relative to self antigens, can be used. Preferably, such sequences are of microbial origin and shown to be immunogenic in HLA-defined or broader populations. In addition to tetanus toxoid (whole or portions, including, but not limited to, portions that are 90%, 80%, 75%, 70%, 60%, 50%, 40%, 30%, 25%, 20%, 15%, 10%, or 5% of a whole toxoid), further examples include, but are not limited to, sequences derived from HBV core, influenza hemagglutinin, Plasmodium circumsporozoite antigen, and HTLV-1 envelope protein, and fragments of these sequences that are 90%, 80%, 75%, 70%, 60%, 50%, 40%, 30%, 25%, 20%, 15%, 10%, or 5% of the respective full-length sequences. In some embodiments, the tetanus toxoid portion is 5% to 90% of a whole toxoid, in other embodiments, the portion is 15% to 80‰ of a whole toxoid, in still other embodiments, the toxoid portion is 25% to 70% of a whole toxoid, in yet other embodiments, the toxoid portion is 35% to 60% of a whole toxoid, in still other embodiments, the toxoid portion is 45% to 55% of a whole toxoid. Similarly, co-administration of a strongly immunogenic B cell epitope (a non-self antigen) with or without a Th epitope (a non-self antigen) with target epitopes (self, tumoral) in a cognate fashion (that is, within the same molecule), can result in improved immune response, or even break of tolerance (T cell) against the therapeutic target, via immune antibody-antigen complexes and bystander T cell help.

### Delivery of the Antigen

While not wanting to be bound by any particular theory, it is thought that T cells do not have a functional memory that is long-lived. Antibody-mediated B-cell memory, on the other hand, appears to have a long-lived effector memory. Thus, delivering an antigen that induces a CTL response is most preferably done over time to keep the patient's immune system appropriately stimulated to attack the target cells. In one approach the presence of antigen is maintained virtually continuously within the lymphatic system to maintain effector CTL function as disclosed in U.S. Patent No. 6,977,074. In another approach T cell memory is repeatedly induced, and re-amplified and reactivated as described in U.S. Patent Application Pub. No. 2005-0079152 A1. While it has been suggested that antigens and adjuvants can be prepared as biodegradable microspheres or liposomes, none of these preparations have thus far provided a CTL response that is useful for attacking cancer cells or pathogens on a long term basis. Preferably, delivery of the antigen is sustained over the desired period of time at a level sufficient to maintain the antigen level to obtain the desired response. In one embodiment, a reservoir having fluid antigen composition can be used to deliver the antigen such that it reaches the animal's lymphatic system. While much of the following discussion focuses on the use of infusion to deliver the antigen it is also possible to use bolus injections directly into the lymphatic system, the number and frequency of which will depend on the persistence of antigen conferred by the particular form and formulation of antigen used.

Ultimately antigen finds its way into the lymphatic system in order to most efficiently stimulate CTL. Delivery of antigen can involve infusion into various compartments of the body, including but not limited to subcutaneous, intravenous, intraperitoneal and intralymphatic, the latter being preferred. While each of these points of infusion results in antigen uptake into the lymphatic system, the relative amounts of antigen needed to induce a beneficial CTL response varies according to the site of infusion. In general, direct infusion of antigen into the lymph system is deemed to be the most efficient means of inducing a CTL response, however, any delivery route can be used. Pump systems are capable of delivering material quantities of antigen in a range that is suitable for inducing a CTL response through delivery to all compartments of the body. CTL stimulation following delivery of antigen via the various routes will vary depending on the properties of different antigens, including factors that influence antigen behavior in the body and its rate of equilibration to (or longevity in) the lymph, such as antigen stability in the body fluid, solubility of antigen in body fluid, binding affinity for HLA and potency as a stimulator of CTL.

In a preferred embodiment, introduction of the antigen is done as directly as possible to the lymphatic system to avoid the destruction of the antigen by metabolism in the body. When introduction of a fluid antigen composition occurs subcutaneously, larger quantities of antigen are needed to assure enough antigen reaches the lymphatic system. Such subcutaneous injection is contemplated herein, depending on factors such as cost, stability of the antigen, how quickly the antigen gets to the lymph system, how well it equilibrates with the lymph, and other factors that the attending doctor or specialist will recognize. Subcutaneous delivery generally can require 100 to 1000 times more antigen than direct delivery to the lymph system. It is preferable, therefore, that the antigen composition is introduced through a device for local administration to the lymphatic system, *e.g.,* the spleen, a lymph node, or a lymph vessel. The device for local administration can be positioned outside the patient or implanted into the patient. In either case, the device can have a reservoir to hold the fluid antigen-containing composition, a pump to transfer the composition, and a transmission channel leading from the reservoir to be directed to the preferred region of administration in the patient's body. In either case it is preferably portable.

For the device positioned outside the patient's body (the external device), there are numerous devices used for delivering insulin to diabetic patients that are useful in delivering antigen according to the embodiments described herein. Generally these devices can be comprised of a reservoir for holding the antigen composition (instead of insulin), a programmable pump to pump the composition out of the reservoir, a transmission channel or line for transmitting the composition, and a means to introduce the composition into the animal's body to ultimately reach the lymphatic system.

Preferably, the reservoir for the antigen composition should be large enough for delivery of the desired amount of antigen over time and easily refillable or replaceable without requiring the user to reinsert the means for introducing the antigen composition to the lymph system.

In preparing the antigen compositions disclosed herein, a composition (preferably aqueous) can be prepared to be compatible with the lymph system and physiologically acceptable to the animal being treated. Relevant considerations include, for example, the physicochemical properties of the antigen, such as the isoelectric point, molecular weight, glycosylation or other post-translational modification, and overall amino acid composition. These properties along with any known behavior of the drug in different solutions (e.g., different buffers, cofactors, etc.) as well as its in vivo behavior can help guide the choice of formulation components. One parameter that impacts all the major degradation pathways is the solution pH. Thus, the initial formulations also assess the pH dependence of the degradation reactions and the mechanism for degradation, which can often be determined from the pH dependence to determine the stability of the protein in each solution. Rapid screening methods usually involve the use of accelerated stability at elevated temperatures (*e.g*., 40° C) using techniques known in the art.

In general the antigen compositions useful in embodiments described herein can be suitable for parenteral injection, in very small quantities. As such a composition should be free of contamination and have a pH compatible with the lymphatic system. However, because very small quantities of the antigenic composition will be delivered it need not be the same pH as blood or lymph, and it need not be aqueous-based. The preferable pH range that is compatible is from about 6.7-7.3 and can be prepared using water for injection to meet USP specifications (see Remington: The Science and Practice of Pharmacy, Nineteenth Edition; Chapters 86-88). For antigens that are less soluble, a suitable cosolvent or surfactant can be used, such as dimethyl sulfoxide (DMSO) or PLURONIC brand surfactants. Generally, a standard saline solution that is buffered with a physiologically acceptable weak acid and its base conjugate, *e.g*., a phosphate or citrate buffering system, will be the basis of the antigen composition. In some cases, a small amount of an antioxidant may be useful to stabilize the composition and prevent oxidation. Factors to consider in preparing the antigen compositions can be found in the 1994 American Chemical Society book entitled Formulation and Delivery of Proteins and Peptides (Acs Symposium Series, No. 567) by Jeffery L. Cleland and Robert Langer (Editor).

For nucleic acid encoded antigens similar considerations can apply, although the variety of physico-chemical properties encountered with polypeptides is absent, so that acceptable formulations will have nearly universal applicability. As seen in Examples 6-10, plasmid DNA in standard phosphate buffered saline (PBS) is an acceptable and effective formulation. In some embodiments, DNA is administered continuously or intermittently at short intervals, from a reservoir worn on, or implanted in, the patient's body. It is preferable that the DNA be maintained in a soluble, stable form at or near body temperature over a period of time measured minimally in days. In such applications where the formulated nucleic acid will be delivered from a reservoir over a period of several days or longer, the stability of the nucleic acid at room or body temperature for that period of time, as well as its continued sterility, take on increased importance. The addition of bacteriostatic agents (*e.g*., benzyl or ethyl alcohol) and chelating agents (*e.g.,* EDTA) is useful toward these ends. Formulations containing about 0.5-2 % ethyl alcohol, 0.25-0.5mM EDTA generally perform well. Such formulations are also appropriate for bolus injections.

Generally the amount of the antigen in the antigen composition will vary from patient to patient and from antigen to antigen, depending on such factors as the activity of the antigen in inducing a response and the flow rate of the lymph through the patient's system. In general the antigen composition may be delivered at a rate of from about 1 to about 500 microliters/hour or about 24 to about 12000 microliters/day. The concentration of the antigen is such that about 0.1 micrograms to about 10,000 micrograms of the antigen will be delivered during 24 hours. The flow rate is based on the knowledge that each minute approximately about 100 to about 1000 microliters of lymph fluid flows through an adult inguinal lymph node. The objective is to maximize local concentration of vaccine formulation in the lymph system. A certain amount of empirical investigation on patients will be necessary to determine the most efficacious level of infusion for a given vaccine preparation in humans.

To introduce the antigen composition into the lymphatic system of the patient the composition is preferably directed to a lymph vessel, lymph node, the spleen, or other appropriate portion of the lymphatic system. Preferably, the composition is directed to a lymph node such as an inguinal or axillary node by inserting a catheter or needle to the node and maintaining the catheter or needle throughout the delivery. Suitable needles or catheters are available made of metal or plastic (*e.g*., polyurethane, polyvinyl chloride (PVC), TEFLON, polyethylene, and the like). In inserting the catheter or needle into the inguinal node for example, the inguinal node is punctured under ultrasonographic control using a Vialon™ Insyte-W™ cannula and catheter of 24G3/4 (Becton Dickinson, USA) which is fixed using Tegaderm™ transparent dressing (Tegaderm™ 1624, 3M, St. Paul, MN 55144, USA). This procedure is generally done by an experienced radiologist. The location of the catheter tip inside the inguinal lymph node is confirmed by injection of a minimal volume of saline, which immediately and visibly increases the size of the lymph node. The latter procedure allows confirmation that the tip is inside the node. This procedure can be performed to ensure that the tip does not slip out of the lymph node and can be repeated on various days after implantation of the catheter. In the event that the tip does slip out of location inside the lymph node, a new catheter can be implanted.

### Formulation and Treatment protocol

There are several approaches to utilizing the combination of TuAAs with DNA vaccines. A first approach is to include all the antigens or epitopes from all the antigens in a given combination into a single DNA expression vector. This approach has the advantages of simplicity for manufacturing and administration to patients. However, in some instances, epitope competition can limit the usefulness of this approach. That is, it is possible that only the most immunogenic epitope will elicit an immune response when a vaccine with several epitopes representing all TuAAs in the combination is given to patients. It is also more difficult to design and construct a DNA vaccine in which all epitopes are expressed at high efficiencies. Nevertheless, because the procedure for treating patients is simple and uniform within each type of cancer, the cost is likely to be lower than for the other approaches described below.

An alternate approach is to include only one antigen or epitopes of one antigen in a DNA expression vector. This approach has the advantages of simplicity in designing and constructing the DNA vector, flexibility, and customized administration to patients. If a large number of individual TuAA vaccines are available, then one can customize treatment for each individual patient based on the TuAA expression profile of his or her tumor. For example, if the standard combination for treating a given type of cancer is TuAA A, B, and C (where A, B, and C designate different tumor associated antigens), but a patient's tumor expresses TuAA A, C, and Z (but not B), then the patient can be treated with separate vaccines for each of A, C, and Z. This flexibility and customizability improves the success rate of immunotherapy because antigen redundancy can be achieved for each patient. However, the procedure of treating the patient can be more complex. For example, delivery using this approach can include a sequential administration scheme (one antigen at a time), or injection into multiple, anatomically separate sites of the patient at about the same time.

Still another approach is to combine epitopes from multiple TuAAs that have similar immunogenicity into a DNA expression vector (more than one vector may be used for some combinations). This approach can have some of the advantages of the above two approaches but also can suffer from the disadvantages of the previous two.

A profile of the antigen expression of a particular tumor can be used to determine which antigen or combination of antigens to use. Exemplary methodology is found in U.S. Patent Application Publication No. 2006-0008468 A1. Specific antigenic combinations of particular benefit in directing an immune response against particular cancers are disclosed in U.S. Patent Application Publication No. 2005-0118186 A1 and PCT Patent Application Pub. No. WO 2004/112825 A1.

Patients that can benefit from such methods of immunization can be recruited using methods to define their MHC protein expression profile and general level of immune responsiveness. In addition, their level of immunity can be monitored using standard techniques in conjunction with access to peripheral blood. Finally, treatment protocols can be adjusted based on the responsiveness to induction or amplification phases and variation in antigen expression. For example, repeated entrainment doses preferably can be administered until a detectable response is obtained, and then administering the amplifying peptide dose(s), rather than amplifying after some set number of entrainment doses. Similarly, scheduled amplifying or maintenance doses of peptide can be discontinued if their effectiveness wanes, antigen-specific regulatory T cell numbers rise, or some other evidence of tolerization is observed, and further entrainment can be administered before resuming amplification with the peptide. The integration of diagnostic techniques to assess and monitor immune responsiveness with methods of immunization is discussed more fully in U.S. Patent Pub No. 2005-0287068 A1.

Combination of active immunotherapies, as disclosed herein, with other treatment modalities can increase the susceptibility of tumoral processes to the elicited immune response and thereby result in increased therapeutic benefit. In some embodiments, the therapeutic benefit is synergistically enhanced. Tumor debulking prior to or during active immunotherapy increases the potential for any particular level of immune response to slow or halt disease progression or to bring about tumor regression or elimination. Additionally, tissue damage, necrosis, or apoptosis initiated with antibody therapy, radiotherapy, biotherapy, chemotherapy, passive immunotherapy (including treatment with mono- and/or polyclonal antibodies, recombinant TCR, and/or adoptive transfer of CTL or other cells of the immune system) or surgery, can facilitate the active immunotherapeutic approach via general inflammation resulting in recruitment of immune effector cells including antigen-specific effectors. In general, any method to induce a transient or more permanent general inflammation within one or multiple tumors / metastatic lesions can facilitate the active immunotherapy. Alternatively or in addition to enabling recruitment of effectors, general inflammation can also increase the susceptibility of target cells to immune mediated attack (*e.g*., as interferons increase expression of target molecules on cancer cells and underlying stroma). Still other strategies to increase susceptibility of tumor cells to immune mediated attack - by providing factors that interfere with the "stress response" or increase target molecules on cancer cells or stromal cells - can synergize with active immunotherapy.

In some embodiments, the terms "a" and "an" and "the" and similar referents used in the context of describing a particular embodiment of the invention (especially in the context of certain of the following claims) may be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (*e*.*g*. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

The following examples are for illustrative purposes only and are not intended to limit the scope of the embodiments in any way.

### Examples

### TuAA analysis and selection of combinations

The presence of TuAAs was measured by Real-Time PGR (RT-PCR). Briefly, total RNA was isolated from tumor specimens by standard methods and cDNA was made with standard reverse transcription procedures. Complementary DNA (cDNA) was amplified with specially designed, gene specific, primers that anneal only to cDNA but not genomic DNA. TuAA expression patterns of 12 ovarian and 7 colorectal tumor specimens were analyzed by RT-PCR. The results are summarized in the Table 5 below.

**Table 5**

| | Total # | PRAME | NY-ESO-1 | SSX-2 | PSMA | MAGE1 | MAGE3 |
|---|---|---|---|---|---|---|---|
| Ovarian | 12 | 12 | 5 | 6 | 6 | 4 | 3 |
| Colorectal | 7 | 5 | 1 | 2 | 5 | 0 | 1 |

### Example 1

### Ovarian Cancer

In the case of ovarian cancer, all samples analyzed were positive for PRAME. Thus the inclusion of PRAME in the combination improves coverage of the cases with ovarian cancer.

In order to achieve antigen redundancy and improve coverage in a large population, combinations of other antigens in addition to PRAME were considered. SSX-2 as well as PSMA were present in 6 of the 12 cases individually, but the combination of SSX-2 and PSMA provided coverage in 9 of 12 cases. Although NY-ESO-1 and SSX-2 were only present in 5 and 6 of the 12 cases, respectively, either NYESO-1 or SSX-2 was detected in 7 of the 12 cases.

Thus, the combination of PRAME, SSX-2, and PSMA or PRAME, NY-ESO-1, and SSX-2 provided preferable coverage and redundancy compared to the combination of PRAME and PSMA or the combination of PRAME and SSX-2. The combination of PRAME, SSX-2, and PSMA provided excellent coverage of cases and good antigen redundancy because the majority of ovarian tumor samples analyzed had at least two of the four TuAA in the combination present. The combination of PRAME, SSX-2, PSMA, and NY-ESO-1 provided more preferred antigen redundancy, and thus, lower possibility of tumor escape.

### Example 2

### Colorectal cancer

In the case of colorectal cancer, PRAME and PSMA were each detected in 5 of the 7 samples analyzed, in 6 of the 7 cases, either PRAME or PSMA was detected. Although SSX-2 was only detected in 2 of 7 cases, both SSX-2-PRAME and SSX2-PSMA combinations increased coverage to 6 of 7. Similarly, although NYESO-1 was detected in only 1 of 7 cases, the combination of NY-ESO-1-PRAME as well as the NYESO-1-PSMA combination increased coverage to 6 of 7. The addition of SSX-2 or NYESO-1 to the PRAME and PSMA combination improved coverage to 7 of 7. Thus, the combination of PRAME, PSMA, and NYESO-1, or the combination of PRAME, PSMA, and SSX-2 provided good coverage of cases and redundancy of antigens for a majority of patients. The combination of PRAME, PSMA, NY-ESO-1, and SSX-2 provided further redundancy.

### Example 3

### Pancreatic Cancer

Real-Time PGR (RT-PCR) was utilized to determine the presence of PRAME, SSX2, NY-ESO-1, and PSMA. Briefly, total RNA was isolated from 5 pancreatic tumor specimens by standard methods and cDNA was made with standard reverse transcription procedures. Complementary DNA (cDNA) was amplified with specially designed, gene-specific primers that anneal only to cDNA but not genomic DNA.

In the pancreatic cancer specimens, the presence of PRAME, NYESO-1, SSX-2, and PSMA was detected in 100%, 40%, 20%, and 100% of the specimens, respectively (see Table 6). Elsewhere, PSMA and over-expression of HER2-/neu were reported to be present in 100% and 21% of pancreatic tumors, respectively (Chang SS et al., Cancer Res 1999, 59:3192; Safran H et al., Am J Clin Oncol. 2001, 24:496). Although over-expression of HER2/neu may render the cancer tissue a preferred target, thus providing some specificity for immunotherapy, low level expression of HER2/neu in normal tissues remains a concern. Thus, the combination of NYESO-1, SSX-2, plus PRAME or PSMA provides excellent coverage and some redundancy for treating pancreatic cancer. Inclusion of both PRAME and PSMA significantly improves redundancy.

**Table 6**

| TAA | PRAME | SSX2 | NY-ESO-1 | PSMA |
|---|---|---|---|---|
| Detection Freq. | 5/5 | 1/5 | 2/5 | 5/5 |
| % positive | 100 | 20 | 40 | 100 |

### Example 4

### Non-small cell lung cancer

For non-small cell lung cancer, the reported presence of NYESO-1, SSX-2, MAGE-3, BAGE, over-expression of Her2/neu, and PSMA was 21, 15, 60, 6, 16, and 100‰, respectively (Scanlan MJ et al., Cancer lett 2000, 150:155; Chang SS et al., Cancer Res 1999, 59:3192; Selvaggi G et al., Cancer 2002, 94:2669). Thus, the combination of NYESO-1, SSX-2, MAGE-3, and PSMA provides coverage and antigen redundancy for the immunotherapy of non-small cell lung cancer.

### Example 5

### Renal cell carcinoma

For renal cell carcinoma, SSX-2, PSMA and PRAME were detected with frequencies of 5, 100 and 40%, respectively (Sahin, U et al., Clin Cancer Res. 2000, 6:3916; Chang SS et al., Urology 2001, 57:801; Neumann E et al., Cancer Res. 1998, 58:4090). Thus, the combination of PSMA and PRAME provides excellent coverage and redundancy for renal cell carcinoma. Adding SSX-2 to the combination of PSMA and PRAME improves redundancy.

### Example 6

### Melanoma

For melanoma, Melan A, Tyrosinase, NYESO-1, and SSX-2 were reported to be present in 92, 92, 41, and 35% of tumor specimens, respectively (Fetsch PA, et al., Cancer 1999, 87:37; Fetsch PA, et al., Cancer 2000, 90:252; Schultz-Thater E et al., Br J Cancer 2000, 83:204; Sahin, U et al., Clin Cancer Res. 2000, 6:3916). Therefore, the combination of Melan A, Tyrosinase, NYESO-1, and SSX-2 provides excellent coverage and antigen redundancy for the immunotherapy of melanoma. Significant redundancy is achieved using tyrosinase and melan-A together, or by combining NY-ESO-1 and SSX-2 with either of tyrosinase or melan-A.

### Example 7

Further studies involving the foregoing tumor types confirmed the observed expression patterns and preferred panels of TuAA. A total of 34 ovarian, 44 colon, 18 renal, and 13 pancreatic tissue samples obtained from various vendors were analyzed for tumor-associated antigen expression using qRTPCR. The results of these assays demonstrated that PRAME and PSMA were expressed frequently (ranging from 68% to 100%) in all four types of tumors studied. NY-ESO-1 and SSX2 were expressed in 20% to 40% of ovarian and pancreatic tumors.

**Table 7:**

| Overall Expression Profiles for Tumor Associated Antigens From RTPCR analysis of Primary Tumors and Metastases | | | | |
|---|---|---|---|---|
| **Tumor-Associated Antigen** | **% Samples Expressing a Given Antigen** | | | |
| | **Ovarian^{a}** | **Renal^{b}** | **Pancreatic^{c}** | **Colorectal^{d}** |
| SSX2 | 36 | 6 | 20 | 8 |
| NY-ESO-1 | 30 | 6 | 40 | 12 |
| PRAME | 97 | 83 | 80 | 76 |
| PSMA | 91 | 100 | 100 | 68 |
| MAGE-1 | 27 | 6 | 33 | 8 |
| MAGE-3 | 30 | 22 | 42 | 20 |
| SCP-1 | 30 | 11 | 0 | 0 |
| CEA | 30 | 0 | 58 | 92 |

| | | | | |
|---|---|---|---|---|
| ^{a} 33 samples (27 primary tumors and 6 metastases) ^{b} 18 samples (18 primary tumors) ^{c} 15 samples (14 primary tumors and 1 metastasis; PSMA on 10 samples) ^{d} 25 samples (13 primary tumors and 12 metastases) | | | | |

### Example 8

### Schedule of immunization with plasmids expressing epitopes from two antigens

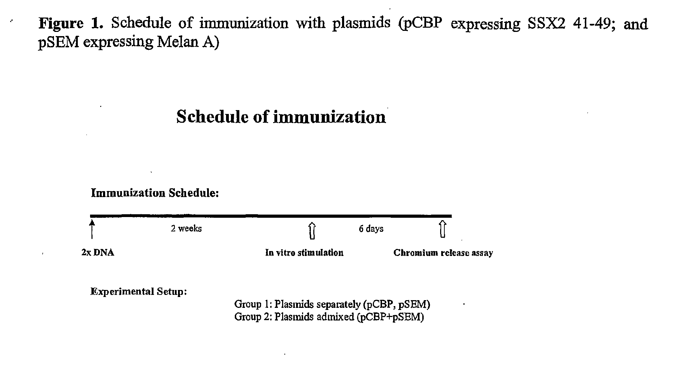

Two groups of HHD mice (n=4) were immunized via intra lymph node injection with either pSEM expressing Melan-A ₂₆₋₃₅A27L (ELA) and pCBP expressing SSX-2₄₁₋₄₉ as a mixture; or with pSEM in the left inguinal lymph node and pCBP in the right inguinal lymph node, twice, at day 0 and 4 as shown in Figure 1. The amount of the plasmid was 25µg/plasmid/dose. Two weeks later, the animals were sacrificed, and cytotoxicity was measured against T2 cells pulsed or not with peptide.

### Example 9

### Co-administration of different vectors carrying distinct antigens

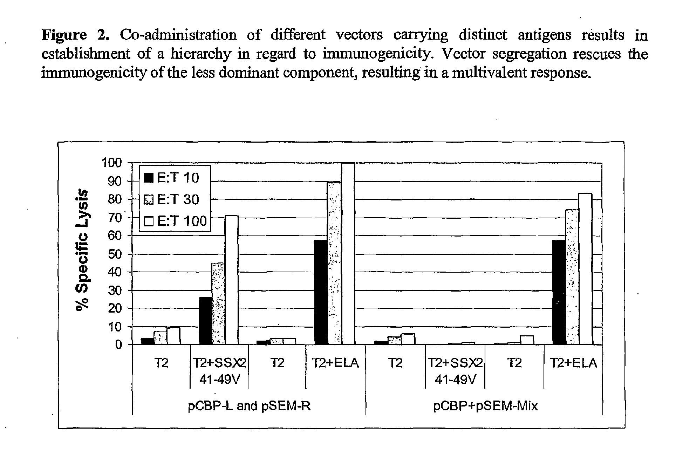

The animals immunized as described in Example 8, were sacrificed and splenocytes from each group pooled and stimulated with the two peptides (ELA or SSX-2₄₁₋₄₉) in parallel. The cytotoxicity was measured by incubation with Cr⁵¹-tagged, peptide loaded T2 target cells. Data in Figure 2 show mean of specific cytotoxicity (n=4/group) against various target cells.

The results show that use of plasmid mixture interferes with the response elicited by pCBP plasmid; however, segregating the two plasmids relative to site of administration rescues the activity of pCBP. Thus, the co-administration of different vectors carrying distinct antigens can result in establishment of a hierarchy with regard to immunogenicity. Vector segregation can rescue the immunogenicity of the less dominant component, resulting in a multivalent response.

### Example 10

### Rescue of Multivalent Response by Addition of Peptide Boost Steps

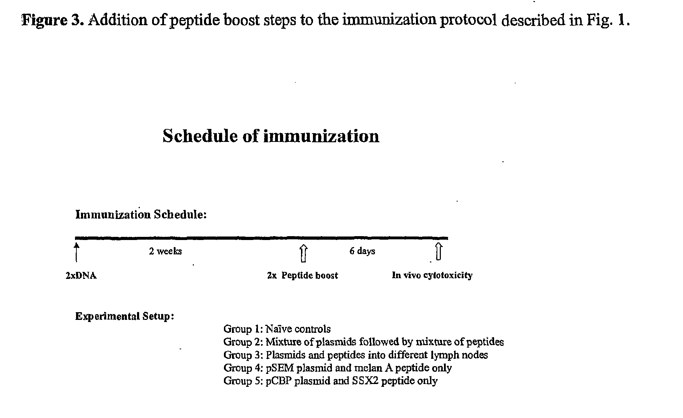

Four groups of HHD mice (n=6) were immunized via intra lymph node injection with either pSEM and pCBP as a mixture; or with pSEM in the left inguinal lymph node and pCBP in the right inguinal lymph node, twice, at day 0 and 4 as shown in Figure 3. As a control, mice were immunized with either pSEM or pCBP plasmid. The amount of the plasmid was 25µg/plasmid/dose. Two weeks later, the animals were boosted with melan A and/or SSX-2 peptides, mirroring the plasmid immunization dose and combination. Two weeks later, the animals were challenged with splenocytes stained with CFSE and loaded or not with Melan A or SSX-2 peptide, for evaluation of in vivo cytotoxicity.

### Example 11

### Peptide amplification rescues the immunogenicity of the less dominant epitope

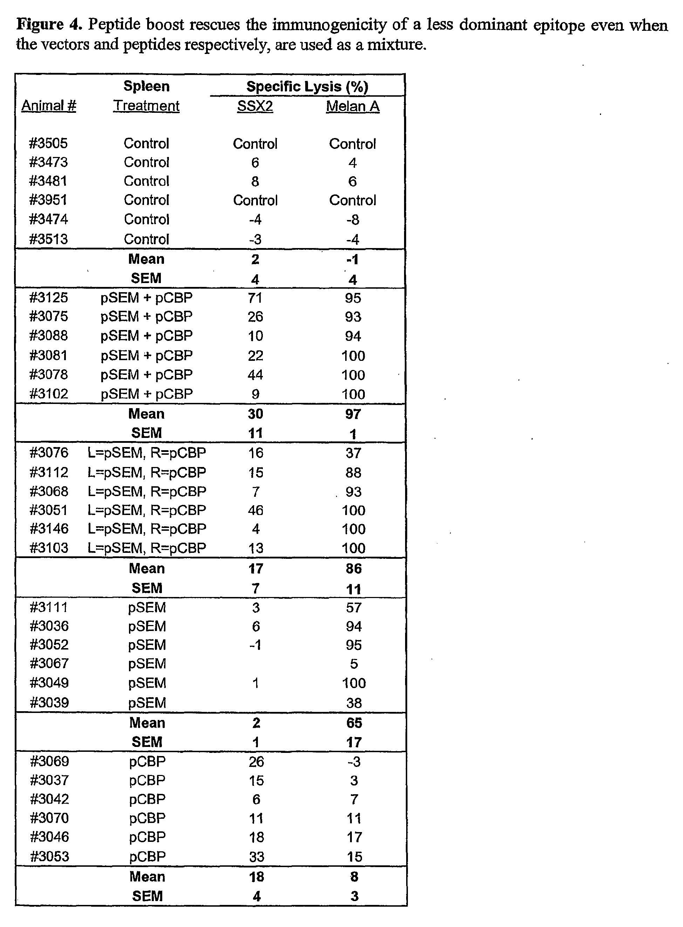

Mice were immunized as described in Example 10 and challenged with HHD littermate splenocytes coated with ELA or SSX-2 peptide, employing a triple peak CFSE *in vivo* cytotoxicity assay that allows the assessment of the specific lysis of two antigen targets simultaneously. Equal numbers of control-CFSE¹⁰, SSX-2₄₁₋₄₉-CFSE^{med}, and ELA-CFSE^{hi} cells were intravenously infused into immunized mice and 18 hours later the mice were sacrificed and target cell elimination was measured in the spleen (Figure 4) by CFSE fluorescence using a flow cytometry. Figure 4 shows the percent specific lysis of the SSX2 and Melan-A antigen targets from individual mice, as well as the mean and SEM for each group.

The results show that immunizing the animals with a mixture of the two vaccines comprising plasmids followed by peptides generated immunity to both antigens and resulted in the highest immune response, representing an average SSX-2 percent specific lysis in the spleen of 30+/-11, and an average Melan-A percent specific lysis of 97+/-1.

### Example 12

### Clinical practice for entrain-and-amplify immunization

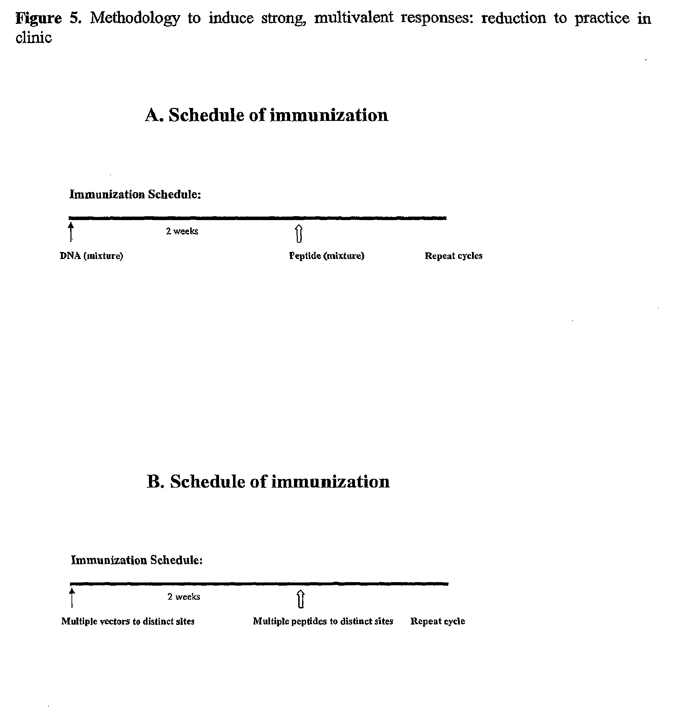

The data in figures 2 and 4 suggest two scenarios for achieving a strong multivalent response in the clinic, shown in Figure 5. In the first scenario (A), use of peptides for boosting restores multivalent immune responses even if plasmids and peptides are used as mixtures. In the second scenario (B), segregation of plasmid and peptide components respectively, allows induction of multivalent immune responses.

### Example 13

### MKC1207: an Entrain-and-Amplify Therapeutic for Melanoma

MKC1207 comprises the plasmid pSEM (described in U.S. Patent Application Pub. No. 2003-0228634 A1, Pub. No. 2004-0132088 A1 and Pub. No. 2004-0203051 A1, in which it is referred to as pMA2M) and peptides corresponding to Melan-A 26-35 and tyrosinase 369-377. The plasmid encodes the A27L analogue of the Melan-A epitope and the native tyrosinase epitope sequence. The plasmids encode both of these epitopes in such a manner that they can be expressed and presented by pAPC. In alternate embodiments of the therapeutic, the peptides can comprise the native sequence or be analogues such as those disclosed in U.S. Patent Application Pub. No. 2006-0057673 A1.

Briefly, the plasmid is administered intranodally to the inguinal lymph nodes as an entraining immunogen. Subsequently, the peptides are administered intranodally, one to the left node, the other to the right as amplifying immunogens. The entrain-and-amplify protocol is described in greater detail in U.S. Patent Application Pub. No. 2005-0079152 A1.

Melanoma patients can be screened according to the methods disclosed herein and MKC1207 administered to patients whose tumor antigen profile includes Melan-A and/or tyrosinase. In a preferred embodiment, the patient's tumor tissue also expresses HLA-A2, particularly HLA-A*0201.

### Example 14

### MKC1106: a Tetravalent Entrain-and-Amplify Therapeutic for Carcinoma

MKC1106 comprises the plasmids pCBP (described in U.S. Patent Application Pub. No. 2003/0228634 A1, Pub. No. 2004-0132088 A1, Pub. No. 2004-0203051 A1) and pRP12; and peptides corresponding to NY-ESO-1 157-165, SSX-2 41-49, PRAME 425-433 and PSMA 288-297. The plasmids encode both of these epitopes in such a manner that they can be expressed and presented by pAPC. In alternate embodiments of the therapeutic, the peptides can comprise the native sequence or be analogues such as those disclosed in U.S. Patent Application Pub. No. 2006-0063913 A1, and Pub No. 2006-0094661 A1, and Pub. No. 2006-0057673 A1.

Briefly, the plasmids are administered intranodally to the inguinal lymph nodes, one to the left side and one to the right, as entraining immunogens. Subsequently the peptides are sequentially administered intranodally, two on separate days to the left node, the other two on separate days to the right as amplifying immunogens. Preferably, the peptides are administered to the same lymph node that received the plasmid encoding the corresponding epitopes. The entrain-and-amplify protocol is described in greater detail in U.S. Patent Application Pub. No. 2005-0079152 A1.

Carcinoma patients, especially those with ovarian, colorectal, pancreatic, or renal cell carcinoma, can be screened according to the methods disclosed herein and MKC1106 administered to patients whose tumor profile includes PRAME, PSMA, NY-ESO-1, and/or SSX-2. The NY-ESO-1 epitope targeted by MKC1106 is also found in LAGE 1a/s, so the presence of this antigen in a profile would also be considered a match. As tumor antigen expression tends to be heterogeneous, any particular tissue sample is likely not to give a complete indication of all the antigens expressed. Thus, it is not necessary that a patient's profile contain all four of the antigens for that patient to be a candidate for treatment with MKC1106. However, preferably the profile contains 2, 3, or 4 of the antigens.

## Claims

1. Use of a PSMA antigen and a PRAME antigen for the manufacture of a medicament for the treatment of pancreatic cancer, wherein the medicament induces an immune response.

2. The use of Claim 1, wherein the medicament further comprises at least one other tumor-associated antigen selected from the group consisting of NY-ESO-1, SSX-2, a MAGE protein, MAGE-3, and Melan-A.

3. The use of Claim 1, wherein a cytolytic T cell response is induced.

4. The use of Claim 1, wherein the medicament further comprises at least one antigen selected from the group consisting of an antigen associated with tumor neovasculature, a growth factor, and a signal transduction protein.

5. The use of Claim 4, wherein the antigen associated with tumor neovasculature is selected from the group consisting of VEGFR2 and Tie-2.

6. The use of Claim 4, wherein said growth factor is VEGF-A.

7. The use of Claim 4, wherein said signal transduction protein is PLK1.

8. The use of Claim 1, wherein the medicament further comprises a stromal cell antigen.

9. The use of Claim 1, wherein the medicament further comprises an extracellular factor antigen.

10. The use of Claim 9, wherein the extra-cellular factor is selected from the group consisting of an autocrine factor, a paracrine factor, a growth factor, chorionic gonadatropin, gastrin, an NF-kB activating factor, VEGF-A, CXCL1, CXCL8, and CCL2.

11. The use of Claim 1, wherein the medicament further comprises means for inducing immunity to a factor that promotes the growth, survival, invasiveness, or metastasis of a tumor.

12. The use of Claim 1, wherein the medicament further comprises a non-self antigen.

13. The use of Claim 12, wherein the non-self antigen comprises a B cell epitope.

14. The use of Claim 12, wherein the non-self antigen comprises a Th epitope.

15. The use of Claim 1, wherein the use is combined with a treatment selected from the group consisting of chemotherapy, radiotherapy, biotherapy, passive immunotherapy, antibody therapy, and surgery.

16. The use of Claim 1, wherein the medicament further comprises a NY-ESO-1 antigen.

17. An immunogenic composition comprising a PSMA antigen and a PRAME antigen for use in the treatment of pancreatic cancer.

18. The immunogenic composition for use according to claim 17, wherein the antigens are provided in the form of 1) whole antigens, 2) fragments of antigens, 3) epitope clusters derived from antigens, 4) epitopes derived from antigens, or 5) nucleic acids encoding any of 1 to 4.

19. The composition for use according to claim 17, further comprising at least one other tumor-associated antigen selected from the group consisting of NY-ESO-1, SSX-2, a MAGE protein, MAGE-3, and Melan-A.

20. The composition for use according to claim 19, wherein the at least one other tumor-associated antigen is selected from NY-ESO-1 and SSX-2.

21. The composition for use according to claim 20, further comprising a growth factor and/or a signal transduction protein.

22. The composition for use according to claim 21, wherein said growth factor is VEGF-A.

23. The composition for use according to claim 21, wherein said signal transduction protein is PLKl.

24. The composition for use according to claim 17, further comprising a neovasculature or other stromal antigen.

25. The composition for use according to claim 24, wherein the antigen associated with tumor neovasculature is selected from the group consisting of VEGFR2 and Tie-2.

26. The composition for use according to claim 17, further comprising an extra-cellular factor antigen.

27. The composition for use according to claim 17, further comprising a non- target antigen.

28. The composition for use according to claim 17, further comprising means for inducing immunity to a factor that promotes the growth, survival, invasiveness, or metastasis of a tumor.

29. The composition for use according to claim 17, further comprising means for inducing bystander help for the tumor-associated antigens.

30. The composition for use according to claim 17, further comprising means for causing inflammation in a tumor lesion.

31. The composition for use according to claim 17, further comprising a NY-ESO-1 antigen.

## Patentansprüche

1. Verwendung eines PSMA-Antigens und eines PRAME-Antigens zur Herstellung eines Medikaments zur Behandlung von Pankreaskrebs, wobei das Medikament eine Immunantwort induziert.

2. Verwendung nach Anspruch 1, wobei das Medikament weiter umfasst mindestens ein weiteres Tumor-assoziiertes Antigen ausgewählt aus der Gruppe bestehend aus NY-ESO-1, SSX-2, einem MAGE-Protein, MAGE-3 und Melan-A.

3. Verwendung nach Anspruch 1, wobei eine zytolytische T-Zellantwort induziert wird.

4. Verwendung nach Anspruch 1, wobei das Medikament weiter umfasst mindestens ein Antigen ausgewählt aus der Gruppe bestehend aus einem Antigen, das mit der Tumorneovaskulatur assoziiert ist, einem Wachstumsfaktor und einem S ignaltransduktionsprotein.

5. Verwendung nach Anspruch 4, wobei das Antigen, das mit der Tumorneovaskulatur assoziiert ist, ausgewählt aus der Gruppe bestehend aus VEGFR2 und Tie-2 ist.

6. Verwendung nach Anspruch 4, wobei der Wachstumsfaktor VEGF-A ist.

7. Verwendung nach Anspruch 4, wobei das Signaltransduktionsprotein PLK1 ist.

8. Verwendung nach Anspruch 1, wobei das Medikament weiter ein Stromazell-Antigen umfasst.

9. Verwendung nach Anspruch 1, wobei das Medikament weiter ein Antigen eines extrazellulären Faktors umfasst.

10. Verwendung nach Anspruch 9, wobei der extrazelluläre Faktor ausgewählt ist aus der Gruppe bestehend aus einem autokrinen Faktor, einem parakrinen Faktor, einem Wachstumsfaktor, Choriongonadotropin, Gastrin, einem NF-κB-aktivierenden Faktor, VEGF-A, CXCL1, CXCL8, und CCL2.

11. Verwendung nach Anspruch 1, wobei das Medikament weiter umfasst Mittel zur Induzierung von Immunität gegenüber einem Faktor, der das Wachstum, das Überleben, die Invasivität oder die Metastasierung eines Tumors fördert.

12. Verwendung nach Anspruch 1, wobei das Medikament weiter ein Nicht-Selbstantigen umfasst.

13. Verwendung nach Anspruch 12, wobei das Nicht-Selbstantigen ein B-Zellepitop umfasst.

14. Verwendung nach Anspruch 12, wobei das Nicht-Selbstantigen ein Th-Epitop umfasst.

15. Verwendung nach Anspruch 1, wobei die Verwendung kombiniert wird mit einer Behandlung ausgewählt aus der Gruppe bestehend aus Chemotherapie, Radiotherapie, Biotherapie, passive Immuntherapie, Antikörpertherapie und Operation.

16. Verwendung nach Anspruch 1, wobei das Medikament weiter ein NY-ESO-1-Antigen umfasst.

17. Immunogene Zusammensetzung umfassend ein PSMA-Antigen und ein PRAME-antigen zu Verwendung in der Behandlung von Pankreaskrebs.

18. Immunogene Zusammensetzung zur Verwendung nach Anspruch 17, wobei die Antigene in Form von 1) ganzen Antigenen, 2) Fragmenten von Antigenen, 3) Epitopklustern, die aus Antigenen stammen, 4) Epitopen, die aus Antigenen stammen, oder 5) Nukleinsäuren, die für eines von 1 bis 4 kodieren, bereitgestellt werden.

19. Zusammensetzung zur Verwendung nach Anspruch 17, weiter umfassend mindestens ein weiteres Tumor-assoziiertes Antigen ausgewählt aus der Gruppe bestehend aus NY-ESO-1, SSX-2, einem MAGE-Protein, MAGE-3, und Melan-A.

20. Zusammensetzung zur Verwendung nach Anspruch 19, wobei das mindestens eine weitere Tumor-assoziierte Antigen ausgewählt ist aus NY-ESO-1 und SSX-2.

21. Zusammensetzung zur Verwendung nach Anspruch 20, weiter umfassend einen Wachstumsfaktor und/oder ein Signaltransduktionsprotein.

22. Zusammensetzung zur Verwendung nach Anspruch 21, wobei der Wachstumsfaktor VEGF-A ist.

23. Zusammensetzung zur Verwendung nach Anspruch 21, wobei das Signaltransduktionsprotein PLK1 ist.

24. Zusammensetzung zur Verwendung nach Anspruch 17, weiter umfassend ein Antigen der Neovaskulatur oder ein anderes Stroma-Antigen.

25. Zusammensetzung zur Verwendung nach Anspruch 24, wobei das Antigen, das mit der Tumorneovaskulatur assoziiert ist, ausgewählt ist aus der Gruppe bestehend aus VEGFR2 und Tie-2.

26. Zusammensetzung zur Verwendung nach Anspruch 17, weiter umfassend ein Antigen eines extrazellulären Faktors.

27. Zusammensetzung zur Verwendung nach Anspruch 17, weiter umfassend ein Nicht-Ziel-Antigen.

28. Zusammensetzung zur Verwendung nach Anspruch 17, weiter umfassend Mittel zur Induzierung von Immunität gegenüber einem Faktor, der das Wachstum, das Überleben, die Invasivität oder die Metastasierung eines Tumors fördert.

29. Zusammensetzung zur Verwendung nach Anspruch 17, weiter umfassend Mittel zur Induzierung einer nebengeordneten Hilfe für die Tumor-assoziierten Antigene.

30. Zusammensetzung zur Verwendung nach Anspruch 17, weiter umfassend Mittel zum Auslösen einer Entzündung in einer Tumorläsion.

31. Zusammensetzung zur Verwendung nach Anspruch 17, weiter umfassend ein NY-ESO-1-Antigen.

## Revendications

1. Utilisation d'un antigène PSMA et d'un antigène PRAME pour la fabrication d'un médicament pour le traitement du cancer du pancréas, sachant que le médicament induit une réponse immunitaire.

2. L'utilisation de la revendication 1, dans laquelle le médicament comprend en outre au moins un autre antigène tumoral sélectionné dans le groupe constitué par NY-ESO-1, SSX-2, une protéine MAGE, MAGE-3, et Melan-A.

3. L'utilisation de la revendication 1, dans laquelle une réponse cytolytique de cellules T est induite.

4. L'utilisation de la revendication 1, dans laquelle le médicament comprend en outre au moins un antigène sélectionné dans le groupe constitué par un antigène associé à la néovasculature tumorale, un facteur de croissance, et une protéine de transduction de signal.

5. L'utilisation de la revendication 4, dans laquelle l'antigène associé à la néovasculature tumorale est sélectionné dans le groupe constitué par VEGFR2 et Tie-2.

6. L'utilisation de la revendication 4, dans laquelle ledit facteur de croissance est VEGF-A.

7. L'utilisation de la revendication 4, dans laquelle ladite protéine de transduction de signal est PKL1.

8. L'utilisation de la revendication 1, dans laquelle le médicament comprend en outre un antigène de cellules stromales.

9. L'utilisation de la revendication 1, dans laquelle le médicament comprend en outre un antigène de facteur extracellulaire.

10. L'utilisation de la revendication 9, dans laquelle le facteur extracellulaire est sélectionné dans le groupe constitué par un facteur autocrine, un facteur paracrine, un facteur de croissance, la gonadatrophine chorionique, la gastrine, un facteur d'activation de NF-kB, VEGF-A, CXCL1, CXCL8, et CCL2.

11. L'utilisation de la revendication 1, dans laquelle le médicament comprend en outre des moyens pour induire une immunité dans un facteur qui promeut la croissance, la survie, le pouvoir envahissant, ou la métastase d'une tumeur.

12. L'utilisation de la revendication 1, dans laquelle le médicament comprend en outre un antigène du non-soi.

13. L'utilisation de la revendication 12, dans laquelle l'antigène du non-soi comprend un épitope de cellule B.

14. L'utilisation de la revendication 12, dans laquelle l'antigène du non-soi comprend un épitope Th.

15. L'utilisation de la revendication 1, dans laquelle l'utilisation est combinée avec un traitement sélectionné dans le groupe constitué par la chimiothérapie, la radiothérapie, la biothérapie, l'immunothérapie passive, la thérapie par anticorps, et la chirurgie.

16. L'utilisation de la revendication 1, dans laquelle le médicament comprend en outre un antigène NY-ESO-1.

17. Composition immunogène comprenant un antigène PSMA et un antigène PRAME, destinée à être utilisée dans le traitement du cancer du pancréas.

18. La composition immunogène destinée à être utilisée selon la revendication 17, dans laquelle les antigènes sont fournis sous la forme de 1) antigènes entiers, 2) fragments d'antigènes, 3) agrégats d'épitopes dérivés d'antigènes, 4) épitopes dérivés d'antigènes, ou 5) acides nucléiques codant l'un quelconque de 1 à 4.

19. La composition destinée à être utilisée selon la revendication 17, comprenant en outre au moins un autre antigène tumoral sélectionné dans le groupe constitué par NY-ESO-1, SSX-2, une protéine MAGE, MAGE-3, et Melan-A.

20. La composition destinée à être utilisée selon la revendication 19, dans laquelle l'au moins un autre antigène tumoral est sélectionné parmi NY-ESO-1 et SSX-2.

21. La composition destinée à être utilisée selon la revendication 20, comprenant en outre un facteur de croissance et/ou une protéine de transduction de signal.

22. La composition destinée à être utilisée selon la revendication 21, dans laquelle ledit facteur de croissance est VEGF-A.

23. La composition destinée à être utilisée selon la revendication 21, dans laquelle ladite protéine de transduction de signal est PLK1.

24. La composition destinée à être utilisée selon la revendication 17, comprenant en outre un antigène de néovasculature ou un autre antigène stromal.

25. La composition destinée à être utilisée selon la revendication 24, dans laquelle l'antigène associé à la néovasculature tumorale est sélectionné dans le groupe constitué par VEGFR2 et Tie-2.

26. La composition destinée à être utilisée selon la revendication 17, comprenant en outre un antigène de facteur extracellulaire.

27. La composition destinée à être utilisée selon la revendication 17, comprenant en outre un antigène non-cible.

28. La composition destinée à être utilisée selon la revendication 17, comprenant en outre des moyens pour induire une immunité dans un facteur qui promeut la croissance, la survie, le pouvoir envahissant, ou la métastase d'une tumeur.

29. La composition destinée à être utilisée selon la revendication 17, comprenant en outre des moyens pour induire une aide de voisinage pour les antigènes tumoraux.

30. La composition destinée à être utilisée selon la revendication 17, comprenant en outre des moyens pour causer une inflammation dans une lésion tumorale.

31. La composition destinée à être utilisée selon la revendication 17, comprenant en outre un antigène NY-ESO-1.


### SEQUENCE LISTING

<110> Chiang, Chih-Sheng Simard, John J.L. Diamond, David C. Bot, Adrian Ion Liu, Xiping
<120> COMBINATIONS OF TUMOR-ASSOCIATED
   ANTIGENS IN COMPOSITIONS FOR VARIOUS TYPES OF CANCERS
<130> MANNK.049VPC
<150> 60/640,598
   <151> 2004-12-29
<160> 26
<170> FastSEQ for windows Version 4.0
<210> 1
   <211> 529
   <212> PRT
   <213> Homo sapiens
<400> 1
<210> 2
   <211> 118
   <212> PRT
   <213> Homo sapiens
<400> 2
   Met Pro Arg Glu Asp Ala His Phe Ile Tyr Gly Tyr Pro Lys Lys Gly
   1 5 10 15
   His Gly His Ser Tyr Thr Thr Ala Glu Glu Ala Ala Gly Ile Gly Ile
   20 25 30
   Leu Thr Val Ile Leu Gly Val Leu Leu Leu Ile Gly Cys Trp Tyr Cys
   35 40 45
   Arg Arg Arg Asn Gly Tyr Arg Ala Leu Met Asp Lys Ser Leu His Val
   50 55 60
   Gly Thr Gln Cys Ala Leu Thr Arg Arg Cys Pro Gln Glu Gly Phe Asp
   65 70 75 80
   His Arg Asp Ser Lys Val Ser Leu Gln Glu Lys Asn Cys Glu Pro Val
   85 90 95
   Val Pro Asn Ala Pro Pro Ala Tyr Glu Lys Leu Ser Ala Glu Gln Ser
   100 105 110
   Pro Pro Pro Tyr Ser Pro
   115
<210> 3
   <211> 223
   <212> PRT
   <213> Homo sapiens
<400> 3
   Met Asn Gly Asp Asp Ala Phe Ala Arg Arg Pro Thr Val Gly Ala Gln
    1               5                  10                  15
   Ile Pro Glu Lys Ile Gln Lys Ala Phe Asp Asp Ile Ala Lys Tyr Phe
               20                  25                  30
   Ser Lys Glu Glu Trp Glu Lys Met Lys Ala Ser Glu Lys Ile Phe Tyr
           35                  40                  45
   Val Tyr Met Lys Arg Lys Tyr Glu Ala Met Thr Lys Leu Gly Phe Lys
       50                  55                  60
   Ala Thr Leu Pro Pro Phe Met Cys Asn Lys Arg Ala Glu Asp Phe Gln
   65                  70                  75                  80
   Gly Asn Asp Leu Asp Asn Asp Pro Asn Arg Gly Asn Gln Val Glu Arg
                   85                  90                  95
   Pro Gln Met Thr Phe Gly Arg Leu Gln Gly Ile Ser Pro Lys Ile Met
               100                 105                 110
   Pro Lys Lys Pro Ala Glu Glu Gly Asn Asp Ser Glu Glu Val Pro Glu
           115                 120                 125
   Ala Ser Gly Pro Gln Asn Asp Gly Lys Glu Leu Cys Pro Pro Gly Lys
       130                 135                 140
   Pro Thr Thr Ser Glu Lys Ile His Glu Arg Ser Gly Asn Arg Glu Ala
   145                 150                 155                 160
   Gln Glu Lys Glu Glu Arg Arg Gly Thr Ala His Arg Trp Ser Ser Gln
                   165                 170                 175
   Asn Thr His Asn Ile Gly Arg Phe Ser Leu Ser Thr Ser Met Gly Ala
               180                 185                 190
   Val His Gly Thr Pro Lys Thr Ile Thr His Asn Arg Asp Pro Lys Gly
           195                 200                 205
   Gly Asn Met Pro Gly Pro Thr Asp Cys Val Arg Glu Asn Ser Trp
       210                 215                 220
<210> 4
   <211> 750
   <212> PRT
   <213> Homo sapiens
<400> 4
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
               260                 265                 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385                 390                 395                 400
   Ser Phe Gly Thr Leu Lys Lys Glu Gly Trp Arg Pro Arg Arg Thr Ile
                   405                 410                 415
   Leu Phe Ala Ser Trp Asp Ala Glu Glu Phe Gly Leu Leu Gly Ser Thr
               420                 425                 430
   Glu Trp Ala Glu Glu Asn Ser Arg Leu Leu Gln Glu Arg Gly Val Ala
           435                 440                 445
   Tyr Ile Asn Ala Asp Ser Ser Ile Glu Gly Asn Tyr Thr Leu Arg Val
       450                 455                 460
   Asp Cys Thr Pro Leu Met Tyr Ser Leu Val His Asn Leu Thr Lys Glu
   465                 470                 475                 480
   Leu Lys Ser Pro Asp Glu Gly Phe Glu Gly Lys Ser Leu Tyr Glu Ser
                   485                 490                 495
   Trp Thr Lys Lys Ser Pro Ser Pro Glu Phe Ser Gly Met Pro Arg Ile
               500                 505                 510
   Ser Lys Leu Gly Ser Gly Asn Asp Phe Glu Val Phe Phe Gln Arg Leu
           515                 520                 525
   Gly Ile Ala Ser Gly Arg Ala Arg Tyr Thr Lys Asn Trp Glu Thr Asn
       530                 535                 540
   Lys Phe Ser Gly Tyr Pro Leu Tyr His Ser Val Tyr Glu Thr Tyr Glu
   545                 550                 555                 560
   Leu Val Glu Lys Phe Tyr Asp Pro Met Phe Lys Tyr His Leu Thr Val
                   565                 570                 575
   Ala Gln Val Arg Gly Gly Met Val Phe Glu Leu Ala Asn Ser Ile Val
               580                 585                 590
   Leu Pro Phe Asp Cys Arg Asp Tyr Ala Val Val Leu Arg Lys Tyr Ala
           595                 600                 605
   Asp Lys Ile Tyr Ser Ile Ser Met Lys His Pro Gln Glu Met Lys Thr
       610                 615                 620
   Tyr Ser Val Ser Phe Asp Ser Leu Phe Ser Ala Val Lys Asn Phe Thr
   625                 630                 635                 640
   Glu Ile Ala Ser Lys Phe Ser Glu Arg Leu Gln Asp Phe Asp Lys Ser
                   645                 650                 655
   Asn Pro Ile Val Leu Arg Met Met Asn Asp Gln Leu Met Phe Leu Glu
               660                 665                 670
   Arg Ala Phe Ile Asp Pro Leu Gly Leu Pro Asp Arg Pro Phe Tyr Arg
           675                 680                 685
   His Val Ile Tyr Ala Pro Ser Ser His Asn Lys Tyr Ala Gly Glu Ser
       690                 695                 700
   Phe Pro Gly Ile Tyr Asp Ala Leu Phe Asp Ile Glu Ser Lys Val Asp
   705                 710                 715                 720
   Pro Ser Lys Ala Trp Gly Glu Val Lys Arg Gln Ile Tyr Val Ala Ala
                   725                 730                 735
   Phe Thr Val Gln Ala Ala Ala Glu Thr Leu Ser Glu Val Ala
               740                 745                 750
<210> 5
   <211> 309
   <212> PRT
   <213> Homo sapiens
<400> 5
   Met Ser Leu Glu Gln Arg Ser Leu His Cys Lys Pro Glu Glu Ala Leu
    1               5                  10                  15
   Glu Ala Gln Gln Glu Ala Leu Gly Leu Val Cys Val Gln Ala Ala Thr
               20                  25                  30
   Ser Ser Ser Ser Pro Leu Val Leu Gly Thr Leu Glu Glu Val Pro Thr
           35                  40                  45
   Ala Gly Ser Thr Asp Pro Pro Gln Ser Pro Gln Gly Ala Ser Ala Phe
       50                  55                  60
   Pro Thr Thr Ile Asn Phe Thr Arg Gln Arg Gln Pro Ser Glu Gly Ser
   65                  70                  75                  80
   Ser Ser Arg Glu Glu Glu Gly Pro Ser Thr Ser Cys Ile Leu Glu Ser
                   85                  90                  95
   Leu Phe Arg Ala Val Ile Thr Lys Lys Val Ala Asp Leu Val Gly Phe
               100                 105                 110
   Leu Leu Leu Lys Tyr Arg Ala Arg Glu Pro Val Thr Lys Ala Glu Met
           115                 120                 125
   Leu Glu Ser Val Ile Lys Asn Tyr Lys His Cys Phe Pro Glu Ile Phe
       130                 135                 140
   Gly Lys Ala Ser Glu Ser Leu Gln Leu Val Phe Gly Ile Asp Val Lys
   145                 150                 155                 160
   Glu Ala Asp Pro Thr Gly His Ser Tyr Val Leu Val Thr Cys Leu Gly
                   165                 170                 175
   Leu Ser Tyr Asp Gly Leu Leu Gly Asp Asn Gln Ile Met Pro Lys Thr
               180                 185                 190
   Gly Phe Leu Ile Ile Val Leu Val Met Ile Ala Met Glu Gly Gly His
           195                 200                 205
   Ala Pro Glu Glu Glu Ile Trp Glu Glu Leu Ser Val Met Glu Val Tyr
       210                 215                 220
   Asp Gly Arg Glu His Ser Ala Tyr Gly Glu Pro Arg Lys Leu Leu Thr
   225                 230                 235                 240
   Gln Asp Leu Val Gln Glu Lys Tyr Leu Glu Tyr Arg Gln Val Pro Asp
                   245                 250                 255
   Ser Asp Pro Ala Arg Tyr Glu Phe Leu Trp Gly Pro Arg Ala Leu Ala
               260                 265                 270
   Glu Thr Ser Tyr Val Lys Val Leu Glu Tyr Val Ile Lys Val Ser Ala
           275                 280                 285
   Arg Val Arg Phe Phe Phe Pro Ser Leu Arg Glu Ala Ala Leu Arg Glu
       290                 295                 300
   Glu Glu Glu Gly Val
   305
<210> 6
   <211> 314
   <212> PRT
   <213> Homo sapiens
<400> 6
   Met Pro Leu Glu Gln Arg Ser Gln His Cys Lys Pro Glu Glu Gly Leu
   1               5                   10                  15
   Glu Ala Arg Gly Glu Ala Leu Gly Leu Val Gly Ala Gln Ala Pro Ala
               20                  25                  30
   Thr Glu Glu Gln Glu Ala Ala Ser Ser Ser Ser Thr Leu Val Glu Val
           35                  40                  45
   Thr Leu Gly Glu Val Pro Ala Ala Glu Ser Pro Asp Pro Pro Gln Ser
       50                  55                  60
   Pro Gln Gly Ala Ser Ser Leu Pro Thr Thr Met Asn Tyr Pro Leu Trp
   65                  70                  75                  80
   Ser Gln Ser Tyr Glu Asp Ser Ser Asn Gln Glu Glu Glu Gly Pro Ser
                   85                  90                  95
   Thr Phe Pro Asp Leu Glu Ser Glu Phe Gln Ala Ala Leu Ser Arg Lys
               100                 105                 110
   Val Ala Glu Leu Val His Phe Leu Leu Leu Lys Tyr Arg Ala Arg Glu
           115                 120                 125
   Pro Val Thr Lys Ala Glu Met Leu Gly Ser Val Val Gly Asn Trp Gln
       130                 135                 140
   Tyr Phe Phe Pro Val Ile Phe Ser Lys Ala Ser Ser Ser Leu Gln Leu
   145                 150                 155                 160
   Val Phe Gly Ile Glu Leu Met Glu Val Asp Pro Ile Gly His Leu Tyr
                   165                 170                 175
   Ile Phe Ala Thr Cys Leu Gly Leu Ser Tyr Asp Gly Leu Leu Gly Asp
               180                 185                 190
   Asn Gln Ile Met Pro Lys Ala Gly Leu Leu Ile Ile Val Leu Ala Ile
           195                 200                 205
   Ile Ala Arg Glu Gly Asp Cys Ala Pro Glu Glu Lys Ile Trp Glu Glu
       210                 215                 220
   Leu Ser Val Leu Glu Val Phe Glu Gly Arg Glu Asp Ser Ile Leu Gly
   225                 230                 235                 240
   Asp Pro Lys Lys Leu Leu Thr Gln His Phe Val Gln Glu Asn Tyr Leu
                   245                 250                 255
   Glu Tyr Arg Gln Val Pro Gly Ser Asp Pro Ala Cys Tyr Glu Phe Leu
               260                 265                 270
   Trp Gly Pro Arg Ala Leu Val Glu Thr Ser Tyr Val Lys Val Leu His
           275                 280                 285
   His Met Val Lys Ile Ser Gly Gly Pro His Ile Ser Tyr Pro Pro Leu
       290                 295                 300
   His Glu Trp Val Leu Arg Glu Gly Glu Glu
   305                 310
<210> 7
   <211> 180
   <212> PRT
   <213> Homo sapiens
<400> 7
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Arg
               180
<210> 8
   <211> 509
   <212> PRT
   <213> Homo sapiens
<400> 8
   Met Glu Arg Arg Arg Leu Trp Gly Ser Ile Gln Ser Arg Tyr Ile Ser
    1               5                  10                  15
   Met Ser Val Trp Thr Ser Pro Arg Arg Leu Val Glu Leu Ala Gly Gln
               20                  25                  30
   Ser Leu Leu Lys Asp Glu Ala Leu Ala Ile Ala Ala Leu Glu Leu Leu
           35                  40                  45
   Pro Arg Glu Leu Phe Pro Pro Leu Phe Met Ala Ala Phe Asp Gly Arg
       50                  55                  60
   His Ser Gln Thr Leu Lys Ala Met Val Gln Ala Trp Pro Phe Thr Cys
   65                  70                  75                  80
   Leu Pro Leu Gly Val Leu Met Lys Gly Gln His Leu His Leu Glu Thr
                   85                  90                  95
   Phe Lys Ala Val Leu Asp Gly Leu Asp Val Leu Leu Ala Gln Glu Val
               100                 105                 110
   Arg Pro Arg Arg Trp Lys Leu Gln Val Leu Asp Leu Arg Lys Asn Ser
           115                 120                 125
   His Gln Asp Phe Trp Thr Val Trp Ser Gly Asn Arg Ala Ser Leu Tyr
       130                 135                 140
   Ser Phe Pro Glu Pro Glu Ala Ala Gln Pro Met Thr Lys Lys Arg Lys
   145                 150                 155                 160
   Val Asp Gly Leu Ser Thr Glu Ala Glu Gln Pro Phe Ile Pro Val Glu
                   165                 170                 175
   Val Leu Val Asp Leu Phe Leu Lys Glu Gly Ala Cys Asp Glu Leu Phe
               180                 185                 190
   Ser Tyr Leu Ile Glu Lys Val Lys Arg Lys Lys Asn Val Leu Arg Leu
           195                 200                 205
   Cys Cys Lys Lys Leu Lys Ile Phe Ala Met Pro Met Gln Asp Ile Lys
       210                 215                 220
   Met Ile Leu Lys Met Val Gln Leu Asp Ser Ile Glu Asp Leu Glu Val
   225                 230                 235                 240
   Thr Cys Thr Trp Lys Leu Pro Thr Leu Ala Lys Phe Ser Pro Tyr Leu
                   245                 250                 255
   Gly Gln Met Ile Asn Leu Arg Arg Leu Leu Leu Ser His Ile His Ala
               260                 265                 270
   Ser Ser Tyr Ile Ser Pro Glu Lys Glu Glu Gln Tyr Ile Ala Gln Phe
           275                 280                 285
   Thr Ser Gln Phe Leu Ser Leu Gln Cys Leu Gln Ala Leu Tyr Val Asp
       290                 295                 300
   Ser Leu Phe Phe Leu Arg Gly Arg Leu Asp Gln Leu Leu Arg His Val
   305                 310                 315                 320
   Met Asn Pro Leu Glu Thr Leu Ser Ile Thr Asn Cys Arg Leu Ser Glu
                   325                 330                 335
   Gly Asp Val Met His Leu Ser Gln Ser Pro Ser Val Ser Gln Leu Ser
               340                 345                 350
   Val Leu Ser Leu Ser Gly Val Met Leu Thr Asp Val Ser Pro Glu Pro
           355                 360                 365
   Leu Gln Ala Leu Leu Glu Arg Ala Ser Ala Thr Leu Gln Asp Leu Val
       370                 375                 380
   Phe Asp Glu Cys Gly Ile Thr Asp Asp Gln Leu Leu Ala Leu Leu Pro
   385                 390                 395                 400
   Ser Leu Ser His Cys Ser Gln Leu Thr Thr Leu Ser Phe Tyr Gly Asn
                   405                 410                 415
   Ser Ile Ser Ile Ser Ala Leu Gln Ser Leu Leu Gln His Leu Ile Gly
               420                 425                 430
   Leu Ser Asn Leu Thr His Val Leu Tyr Pro Val Pro Leu Glu Ser Tyr
           435                 440                 445
   Glu Asp Ile His Gly Thr Leu His Leu Glu Arg Leu Ala Tyr Leu His
       450                 455                 460
   Ala Arg Leu Arg Glu Leu Leu Cys Glu Leu Gly Arg Pro Ser Met Val
   465                 470                 475                 480
   Trp Leu Ser Ala Asn Pro Cys Pro His Cys Gly Asp Arg Thr Phe Tyr
                   485                 490                 495
   Asp Pro Glu Pro Ile Leu Cys Pro Cys Phe Met Pro Asn
               500                 505
<210> 9
   <211> 1255
   <212> PRT
   <213> Homo sapiens
<400> 9
<210> 10
   <211> 622
   <212> PRT
   <213> Homo sapiens
<400> 10
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385 390 395 400
   Val Asn Lys Gly His Glu Met Ser Pro Gln Val Ala Thr Leu Ile Asp
   405 410 415
   Arg Phe Val Lys Gly Arg Gly Gln Leu Asp Lys Asp Thr Leu Asp Thr
   420 425 430
   Leu Thr Ala Phe Tyr Pro Gly Tyr Leu Cys Ser Leu Ser Pro Glu Glu
   435 440 445
   Leu Ser Ser Val Pro Pro Ser Ser Ile Trp Ala Val Arg Pro Gln Asp
   450 455 460
   Leu Asp Thr Cys Asp Pro Arg Gln Leu Asp Val Leu Tyr Pro Lys Ala
   465 470 475 480
   Arg Leu Ala Phe Gln Asn Met Asn Gly Ser Glu Tyr Phe Val Lys Ile
   485 490 495
   Gln Ser Phe Leu Gly Gly Ala Pro Thr Glu Asp Leu Lys Ala Leu Ser
   500 505 510
   Gln Gln Asn Val Ser Met Asp Leu Ala Thr Phe Met Lys Leu Arg Thr
   515 520 525
   Asp Ala Val Leu Pro Leu Thr Val Ala Glu Val Gln Lys Leu Leu Gly
   530 535 540
   Pro His Val Glu Gly Leu Lys Ala Glu Glu Arg His Arg Pro Val Arg
   545 550 555 560
   Asp Trp Ile Leu Arg Gln Arg Gln Asp Asp Leu Asp Thr Leu Gly Leu
   565 570 575
   Gly Leu Gln Gly Gly Ile Pro Asn Gly Tyr Leu Val Leu Asp Leu Ser
   580 585 590
   Met Gln Glu Ala Leu Ser Gly Thr Pro Cys Leu Leu Gly Pro Gly Pro
   595 600 605
   Val Leu Thr Val Leu Ala Leu Leu Leu Ala Ser Thr Leu Ala
   610 615 620
<210> 11
   <211> 630
   <212> PRT
   <213> Homo sapiens
<400> 11
   Met Ala Leu Pro Thr Ala Arg Pro Leu Leu Gly Ser Cys Gly Thr Pro
    1               5                  10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
   245 250 255
   Gln Pro Ile Ile Arg Ser Ile Pro Gln Gly Ile Val Ala Ala Trp Arg
   260 265 270
   Gln Arg Ser Ser Arg Asp Pro Ser Trp Arg Gln Pro Glu Arg Thr Ile
   275 280 285
   Leu Arg Pro Arg Phe Arg Arg Glu Val Glu Lys Thr Ala Cys Pro Ser
   290 295 300
   Gly Lys Lys Ala Arg Glu Ile Asp Glu Ser Leu Ile Phe Tyr Lys Lys
   305 310 315 320
   Trp Glu Leu Glu Ala Cys Val Asp Ala Ala Leu Leu Ala Thr Gln Met
   325 330 335
   Asp Arg Val Asn Ala Ile Pro Phe Thr Tyr Glu Gln Leu Asp Val Leu
   340 345 350
   Lys His Lys Leu Asp Glu Leu Tyr Pro Gln Gly Tyr Pro Glu Ser Val
   355 360 365
   Ile Gln His Leu Gly Tyr Leu Phe Leu Lys Met Ser Pro Glu Asp Ile
   370 375 380
   Arg Lys Trp Asn Val Thr Ser Leu Glu Thr Leu Lys Ala Leu Leu Glu
   385 390 395 400
   Val Asn Lys Gly His Glu Met Ser Pro Gln Ala Pro Arg Arg Pro Leu
   405 410 415
   Pro Gln Val Ala Thr Leu Ile Asp Arg Phe Val Lys Gly Arg Gly Gln
   420 425 430
   Leu Asp Lys Asp Thr Leu Asp Thr Leu Thr Ala Phe Tyr Pro Gly Tyr
   435 440 445
   Leu Cys Ser Leu Ser Pro Glu Glu Leu Ser Ser Val Pro Pro Ser Ser
   450 455 460
   Ile Trp Ala Val Arg Pro Gln Asp Leu Asp Thr Cys Asp Pro Arg Gln
   465 470 475 480
   Leu Asp Val Leu Tyr Pro Lys Ala Arg Leu Ala Phe Gln Asn Met Asn
   485 490 495
   Gly Ser Glu Tyr Phe Val Lys Ile Gln Ser Phe Leu Gly Gly Ala Pro
   500 505 510
   Thr Glu Asp Leu Lys Ala Leu Ser Gln Gln Asn Val Ser Met Asp Leu
   515 520 525
   Ala Thr Phe Met Lys Leu Arg Thr Asp Ala Val Leu Pro Leu Thr Val
   530 535 540
   Ala Glu Val Gln Lys Leu Leu Gly Pro His Val Glu Gly Leu Lys Ala
   545 550 555 560
   Glu Glu Arg His Arg Pro Val Arg Asp Trp Ile Leu Arg Gln Arg Gln
   565 570 575
   Asp Asp Leu Asp Thr Leu Gly Leu Gly Leu Gln Gly Gly Ile Pro Asn
   580 585 590
   Gly Tyr Leu Val Leu Asp Leu Ser Met Gln Glu Ala Leu Ser Gly Thr
   595 600 605
   Pro Cys Leu Leu Gly Pro Gly Pro Val Leu Thr Val Leu Ala Leu Leu
   610 615 620
   Leu Ala Ser Thr Leu Ala
   625 630
<210> 12
   <211> 232
   <212> PRT
   <213> Homo sapiens
<400> 12
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Lys Lys Ser Val
   130 135 140
   Arg Gly Lys Gly Lys Gly Gln Lys Arg Lys Arg Lys Lys Ser Arg Tyr
   145 150 155 160
   Lys Ser Trp Ser Val Tyr Val Gly Ala Arg Cys Cys Leu Met Pro Trp
   165 170 175
   Ser Leu Pro Gly Pro His Pro Cys Gly Pro Cys Ser Glu Arg Arg Lys
   180 185 190
   His Leu Phe Val Gln Asp Pro Gln Thr Cys Lys Cys Ser Cys Lys Asn
   195 200 205
   Thr Asp Ser Arg Cys Lys Ala Arg Gln Leu Glu Leu Asn Glu Arg Thr
   210 215 220
   Cys Arg Cys Asp Lys Pro Arg Arg
   225 230
<210> 13
   <211> 603
   <212> PRT
   <213> Homo sapiens
<400> 13
   Met Ser Ala Ala Val Thr Ala Gly Lys Leu Ala Arg Ala Pro Ala Asp
   1 5 10 15
   Pro Gly Lys Ala Gly Val Pro Gly Val Ala Ala Pro Gly Ala Pro Ala
   20 25 30
   Ala Ala Pro Pro Ala Lys Glu Ile Pro Glu Val Leu Val Asp Pro Arg
   35 40 45
   Ser Arg Arg Arg Tyr Val Arg Gly Arg Phe Leu Gly Lys Gly Gly Phe
   50 55 60
   Ala Lys Cys Phe Glu Ile Ser Asp Ala Asp Thr Lys Glu Val Phe Ala
   65 70 75 80
   Gly Lys Ile Val Pro Lys Ser Leu Leu Leu Lys Pro His Gln Arg Glu
   85 90 95
   Lys Met Ser Met Glu Ile Ser Ile His Arg Ser Leu Ala His Gln His
   100 105 110
   Val Val Gly Phe His Gly Phe Phe Glu Asp Asn Asp Phe Val Phe Val
   115 120 125
   Val Leu Glu Leu Cys Arg Arg Arg Ser Leu Leu Glu Leu His Lys Arg
   130 135 140
   Arg Lys Ala Leu Thr Glu Pro Glu Ala Arg Tyr Tyr Leu Arg Gln Ile
   145 150 155 160
   Val Leu Gly Cys Gln Tyr Leu His Arg Asn Arg Val Ile His Arg Asp
   165 170 175
   Leu Lys Leu Gly Asn Leu Phe Leu Asn Glu Asp Leu Glu Val Lys Ile
   180 185 190
   Gly Asp Phe Gly Leu Ala Thr Lys Val Glu Tyr Asp Gly Glu Arg Lys
   195 200 205
   Lys Thr Leu Cys Gly Thr Pro Asn Tyr Ile Ala Pro Glu Val Leu Ser
   210 215 220
   Lys Lys Gly His Ser Phe Glu Val Asp Val Trp Ser Ile Gly Cys Ile
   225 230 235 240
   Met Tyr Thr Leu Leu Val Gly Lys Pro Pro Phe Glu Thr Ser Cys Leu
   245 250 255
   Lys Glu Thr Tyr Leu Arg Ile Lys Lys Asn Glu Tyr Ser Ile Pro Lys
   260 265 270
   His Ile Asn Pro Val Ala Ala Ser Leu Ile Gln Lys Met Leu Gln Thr
   275 280 285
   Asp Pro Thr Ala Arg Pro Thr Ile Asn Glu Leu Leu Asn Asp Glu Phe
   290 295 300
   Phe Thr Ser Gly Tyr Ile Pro Ala Arg Leu Pro Ile Thr Cys Leu Thr
   305 310 315 320
   Ile Pro Pro Arg Phe Ser Ile Ala Pro Ser Ser Leu Asp Pro Ser Asn
                   325                 330                 335
   Arg Lys Pro Leu Thr Val Leu Asn Lys Gly Leu Glu Asn Pro Leu Pro
               340                 345                 350
   Glu Arg Pro Arg Glu Lys Glu Glu Pro Val Val Arg Glu Thr Gly Glu
           355                 360                 365
   Val Val Asp Cys His Leu Ser Asp Met Leu Gln Gln Leu His Ser Val
       370                 375                 380
   Asn Ala Ser Lys Pro Ser Glu Arg Gly Leu Val Arg Gln Glu Glu Ala
   385                 390                 395                 400
   Glu Asp Pro Ala Cys Ile Pro Ile Phe Trp Val Ser Lys Trp Val Asp
                   405                 410                 415
   Tyr Ser Asp Lys Tyr Gly Leu Gly Tyr Gln Leu Cys Asp Asn Ser Val
               420                 425                 430
   Gly Val Leu Phe Asn Asp Ser Thr Arg Leu Ile Leu Tyr Asn Asp Gly
           435                 440                 445
   Asp Ser Leu Gln Tyr Ile Glu Arg Asp Gly Thr Glu Ser Tyr Leu Thr
       450                 455                 460
   Val Ser Ser His Pro Asn Ser Leu Met Lys Lys Ile Thr Leu Leu Lys
   465                 470                 475                 480
   Tyr Phe Arg Asn Tyr Met Ser Glu His Leu Leu Lys Ala Gly Ala Asn
                   485                 490                 495
   Ile Thr Pro Arg Glu Gly Asp Glu Leu Ala Arg Leu Pro Tyr Leu Arg
               500                 505                 510
   Thr Trp Phe Arg Thr Arg Ser Ala Ile Ile Leu His Leu Ser Asn Gly
           515                 520                 525
   Ser Val Gln Ile Asn Phe Phe Gln Asp His Thr Lys Leu Ile Leu Cys
       530                 535                 540
   Pro Leu Met Ala Ala Val Thr Tyr Ile Asp Glu Lys Arg Asp Phe Arg
   545                 550                 555                 560
   Thr Tyr Arg Leu Ser Leu Leu Glu Glu Tyr Gly Cys Cys Lys Glu Leu
                   565                 570                 575
   Ala Ser Arg Leu Arg Tyr Ala Arg Thr Met Val Asp Lys Leu Leu Ser
               580                 585                 590
   Ser Arg Ser Ala Ser Asn Arg Leu Lys Ala Ser
           595                 600
<210> 14
   <211> 2384
   <212> DNA
   <213> Homo sapiens
<400> 14
   tattgagttc ttcaaacatt gtagcctctt tatggtctct gagaaataac taccttaaac 60
   ccataatctt taatacttcc taaactttct taataagaga agctctattc ctgacactac 120
   ctctcatttg caaggtcaaa tcatcattag ttttgtagtc tattaactgg gtttgcttag 180
   gtcaggcatt attattacta accttattgt taatattcta accataagaa ttaaactatt 240
   aatggtgaat agagtttttc actttaacat aggcctatcc cactggtggg atacgagcca 300
   attcgaaaga aaagtcagtc atgtgctttt cagaggatga aagcttaaga taaagactaa 360
   aagtgtttga tgctggaggt gggagtggta ttatataggt ctcagccaag acatgtgata 420
   atcactgtag tagtagctgg aaagagaaat ctgtgactcc aattagccag ttcctgcaga 480
   ccttgtgagg actagaggaa gaatgctcct ggctgttttg tactgcctgc tgtggagttt 540
   ccagacctcc gctggccatt tccctagagc ctgtgtctcc tctaagaacc tgatggagaa 600
   ggaatgctgt ccaccgtgga gcggggacag gagtccctgt ggccagcttt caggcagagg 660
   ttcctgtcag aatatccttc tgtccaatgc accacttggg cctcaatttc ccttcacagg 720
   ggtggatgac cgggagtcgt ggccttccgt cttttataat aggacctgcc agtgctctgg 780
   caacttcatg ggattcaact gtggaaactg caagtttggc ttttggggac caaactgcac 840
   agagagacga ctcttggtga gaagaaacat cttcgatttg agtgccccag agaaggacaa 900
   attttttgcc tacctcactt tagcaaagca taccatcagc tcagactatg tcatccccat 960
   agggacctat ggccaaatga aaaatggatc aacacccatg tttaacgaca tcaatattta 1020
   tgacctcttt gtctggatgc attattatgt gtcaatggat gcactgcttg ggggatctga 1080
   aatctggaga gacattgatt ttgcccatga agcaccagct tttctgcctt ggcatagact 1140
   cttcttgttg cggtgggaac aagaaatcca gaagctgaca ggagatgaaa acttcactat 1200
   tccatattgg gactggcggg atgcagaaaa gtgtgacatt tgcacagatg agtacatggg 1260
   aggtcagcac cccacaaatc ctaacttact cagcccagca tcattcttct cctcttggca 1320
   gattgtctgt agccgattgg aggagtacaa cagccatcag tctttatgca atggaacgcc 1380
   cgagggacct ttacggcgta atcctggaaa ccatgacaaa tccagaaccc caaggctccc 1440
   ctcttcagct gatgtagaat tttgcctgag tttgacccaa tatgaatctg gttccatgga 1500
   taaagctgcc aatttcagct ttagaaatac actggaagga tttgctagtc cacttactgg 1560
   gatagcggat gcctctcaaa gcagcatgca caatgccttg cacatctata tgaatggaac 1620
   aatgtcccag gtacagggat ctgccaacga tcctatcttc cttcttcacc atgcatttgt 1680
   tgacagtatt tttgagcagt ggctccgaag gcaccgtcct cttcaagaag tttatccaga 1740
   agccaatgca cccattggac ataaccggga atcctacatg gttcctttta taccactgta 1800
   cagaaatggt gatttcttta tttcatccaa agatctgggc tatgactata gctatctaca 1860
   agattcagac ccagactctt ttcaagacta cattaagtcc tatttggaac aagcgagtcg 1920
   gatctggtca tggctccttg gggcggcgat ggtaggggcc gtcctcactg ccctgctggc 1980
   agggcttgtg agcttgctgt gtcgtcacaa gagaaagcag cttcctgaag aaaagcagcc 2040
   actcctcatg gagaaagagg attaccacag cttgtatcag agccatttat aaaaggctta 2100
   ggcaatagag tagggccaaa aagcctgacc tcactctaac tcaaagtaat gtccaggttc 2160
   ccagagaata tctgctggta tttttctgta aagaccattt gcaaaattgt aacctaatac 2220
   aaagtgtagc cttcttccaa ctcaggtaga acacacctgt ctttgtcttg ctgttttcac 2280
   tcagcccttt taacattttc ccctaagccc atatgtctaa ggaaaggatg ctatttggta 2340
   atgaggaact gttatttgta tgtgaattaa agtgctctta tttt                  2384
<210> 15
   <211> 1524
   <212> DNA
   <213> Homo sapiens
<400> 15
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg 1524
<210> 16
   <211> 1466
   <212> DNA
   <213> Homo sapiens
<400> 16
   gcatgctctg actttctctc tctttcgatt cttccatact cagagtacgc acggtctgat 60
   tttctctttg gattcttcca aaatcagagt cagactgctc ccggtgccat gaacggagac 120
   gacgcctttg caaggagacc cacggttggt gctcaaatac cagagaagat ccaaaaggcc 180
   ttcgatgata ttgccaaata cttctctaag gaagagtggg aaaagatgaa agcctcggag 240
   aaaatcttct atgtgtatat gaagagaaag tatgaggcta tgactaaact aggtttcaag 300
   gccaccctcc cacctttcat gtgtaataaa cgggccgaag acttccaggg gaatgatttg 360
   gataatgacc ctaaccgtgg gaatcaggtt gaacgtcctc agatgacttt cggcaggctc 420
   cagggaatct ccccgaagat catgcccaag aagccagcag aggaaggaaa tgattcggag 480
   gaagtgccag aagcatctgg cccacaaaat gatgggaaag agctgtgccc cccgggaaaa 540
   ccaactacct ctgagaagat tcacgagaga tctggaaata gggaggccca agaaaaggaa 600
   gagagacgcg gaacagctca tcggtggagc agtcagaaca cacacaacat tggtcgattc 660
   agtttgtcaa cttctatggg tgcagttcat ggtaccccca aaacaattac acacaacagg 720
   gacccaaaag gggggaacat gcctggaccc acagactgcg tgagagaaaa cagctggtga 780
   tttatgaaga gatcagcgac cctgaggaag atgacgagta actcccctca gggatacgac 840
   acatgcccat gatgagaagc agaacgtggt gacctttcac gaacatgggc atggctgcgg 900
   acccctcgtc atcaggtgca tagcaagtga aagcaagtgt tcacaacagt gaaaagttga 960
   gcgtcatttt tcttagtgtg ccaagagttc gatgttagcg tttacgttgt attttcttac 1020
   actgtgtcat tctgttagat actaacattt tcattgatga gcaagacata cttaatgcat 1080
   attttggttt gtgtatccat gcacctacct tagaaaacaa gtattgtcgg ttacctctgc 1140
   atggaacagc attaccctcc tctctcccca gatgtgacta ctgagggcag ttctgagtgt 1200
   ttaatttcag attttttcct ctgcatttac acacacacgc acacaaacca caccacacac 1260
   acacacacac acacacacac acacacacac acacaccaag taccagtata agcatctgcc 1320
   atctgctttt cccattgcca tgcgtcctgg tcaagctccc ctcactctgt ttcctggtca 1380
   gcatgtactc ccctcatccg attcccctgt agcagtcact gacagttaat aaacctttgc 1440
   aaacgttcaa aaaaaaaaaa aaaaaa 1466
<210> 17
   <211> 2653
   <212> DNA
   <213> Homo sapiens
<400> 17
   ctcaaaaggg gccggatttc cttctcctgg aggcagatgt tgcctctctc tctcgctcgg 60
   attggttcag tgcactctag aaacactgct gtggtggaga aactggaccc caggtctgga 120
   gcgaattcca gcctgcaggg ctgataagcg aggcattagt gagattgaga gagactttac 180
   cccgccgtgg tggttggagg gcgcgcagta gagcagcagc acaggcgcgg gtcccgggag 240
   gccggctctg ctcgcgccga gatgtggaat ctccttcacg aaaccgactc ggctgtggcc 300
   accgcgcgcc gcccgcgctg gctgtgcgct ggggcgctgg tgctggcggg tggcttcttt 360
   ctcctcggct tcctcttcgg gtggtttata aaatcctcca atgaagctac taacattact 420
   ccaaagcata atatgaaagc atttttggat gaattgaaag ctgagaacat caagaagttc 480
   ttatataatt ttacacagat accacattta gcaggaacag aacaaaactt tcagcttgca 540
   aagcaaattc aatcccagtg gaaagaattt ggcctggatt ctgttgagct agcacattat 600
   gatgtcctgt tgtcctaccc aaataagact catcccaact acatctcaat aattaatgaa 660
   gatggaaatg agattttcaa cacatcatta tttgaaccac ctcctccagg atatgaaaat 720
   gtttcggata ttgtaccacc tttcagtgct ttctctcctc aaggaatgcc agagggcgat 780
   ctagtgtatg ttaactatgc acgaactgaa gacttcttta aattggaacg ggacatgaaa 840
   atcaattgct ctgggaaaat tgtaattgcc agatatggga aagttttcag aggaaataag 900
   gttaaaaatg cccagctggc aggggccaaa ggagtcattc tctactccga ccctgctgac 960
   tactttgctc ctggggtgaa gtcctatcca gatggttgga atcttcctgg aggtggtgtc 1020
   cagcgtggaa atatcctaaa tctgaatggt gcaggagacc ctctcacacc aggttaccca 1080
   gcaaatgaat atgcttatag gcgtggaatt gcagaggctg ttggtcttcc aagtattcct 1140
   gttcatccaa ttggatacta tgatgcacag aagctcctag aaaaaatggg tggctcagca 1200
   ccaccagata gcagctggag aggaagtctc aaagtgccct acaatgttgg acctggcttt 1260
   actggaaact tttctacaca aaaagtcaag atgcacatcc actctaccaa tgaagtgaca 1320
   agaatttaca atgtgatagg tactctcaga ggagcagtgg aaccagacag atatgtcatt 1380
   ctgggaggtc accgggactc atgggtgttt ggtggtattg accctcagag tggagcagct 1440
   gttgttcatg aaattgtgag gagctttgga acactgaaaa aggaagggtg gagacctaga 1500
   agaacaattt tgtttgcaag ctgggatgca gaagaatttg gtcttcttgg ttctactgag 1560
   tgggcagagg agaattcaag actccttcaa gagcgtggcg tggcttatat taatgctgac 1620
   tcatctatag aaggaaacta cactctgaga gttgattgta caccgctgat gtacagcttg 1680
   gtacacaacc taacaaaaga gctgaaaagc cctgatgaag gctttgaagg caaatctctt 1740
   tatgaaagtt ggactaaaaa aagtccttcc ccagagttca gtggcatgcc caggataagc 1800
   aaattgggat ctggaaatga ttttgaggtg ttcttccaac gacttggaat tgcttcaggc 1860
   agagcacggt atactaaaaa ttgggaaaca aacaaattca gcggctatcc actgtatcac 1920
   agtgtctatg aaacatatga gttggtggaa aagttttatg atccaatgtt taaatatcac 1980
   ctcactgtgg cccaggttcg aggagggatg gtgtttgagc tagccaattc catagtgctc 2040
   ccttttgatt gtcgagatta tgctgtagtt ttaagaaagt atgctgacaa aatctacagt 2100
   atttctatga aacatccaca ggaaatgaag acatacagtg tatcatttga ttcacttttt 2160
   tctgcagtaa agaattttac agaaattgct tccaagttca gtgagagact ccaggacttt 2220
   gacaaaagca acccaatagt attaagaatg atgaatgatc aactcatgtt tctggaaaga 2280
   gcatttattg atccattagg gttaccagac aggccttttt ataggcatgt catctatgct 2340
   ccaagcagcc acaacaagta tgcaggggag tcattcccag gaatttatga tgctctgttt 2400
   gatattgaaa gcaaagtgga cccttccaag gcctggggag aagtgaagag acagatttat 2460
   gttgcagcct tcacagtgca ggcagctgca gagactttga gtgaagtagc ctaagaggat 2520
   tctttagaga atccgtattg aatttgtgtg gtatgtcact cagaaagaat cgtaatgggt 2580
   atattgataa attttaaaat tggtatattt gaaataaagt tgaatattat atataaaaaa 2640
   aaaaaaaaaa aaa                                                    2653
<210> 18
   <211> 2420
   <212> DNA
   <213> Homo sapiens
<400> 18
   ggatccaggc cctgccagga aaaatataag ggccctgcgt gagaacagag ggggtcatcc 60
   actgcatgag agtggggatg tcacagagtc cagcccaccc tcctggtagc actgagaagc 120
   cagggctgtg cttgcggtct gcaccctgag ggcccgtgga ttcctcttcc tggagctcca 180
   ggaaccaggc agtgaggcct tggtctgaga cagtatcctc aggtcacaga gcagaggatg 240
   cacagggtgt gccagcagtg aatgtttgcc ctgaatgcac accaagggcc ccacctgcca 300
   caggacacat aggactccac agagtctggc ctcacctccc tactgtcagt cctgtagaat 360
   cgacctctgc tggccggctg taccctgagt accctctcac ttcctccttc aggttttcag 420
   gggacaggcc aacccagagg acaggattcc ctggaggcca cagaggagca ccaaggagaa 480
   gatctgtaag taggcctttg ttagagtctc caaggttcag ttctcagctg aggcctctca 540
   cacactccct ctctccccag gcctgtgggt cttcattgcc cagctcctgc ccacactcct 600
   gcctgctgcc ctgacgagag tcatcatgtc tcttgagcag aggagtctgc actgcaagcc 660
   tgaggaagcc cttgaggccc aacaagaggc cctgggcctg gtgtgtgtgc aggctgccac 720
   ctcctcctcc tctcctctgg tcctgggcac cctggaggag gtgcccactg ctgggtcaac 780
   agatcctccc cagagtcctc agggagcctc cgcctttccc actaccatca acttcactcg 840
   acagaggcaa cccagtgagg gttccagcag ccgtgaagag gaggggccaa gcacctcttg 900
   tatcctggag tccttgttcc gagcagtaat cactaagaag gtggctgatt tggttggttt 960
   tctgctcctc aaatatcgag ccagggagcc agtcacaaag gcagaaatgc tggagagtgt 1020
   catcaaaaat tacaagcact gttttcctga gatcttcggc aaagcctctg agtccttgca 1080
   gctggtcttt ggcattgacg tgaaggaagc agaccccacc ggccactcct atgtccttgt 1140
   cacctgccta ggtctctcct atgatggcct gctgggtgat aatcagatca tgcccaagac 1200
   aggcttcctg ataattgtcc tggtcatgat tgcaatggag ggcggccatg ctcctgagga 1260
   ggaaatctgg gaggagctga gtgtgatgga ggtgtatgat gggagggagc acagtgccta 1320
   tggggagccc aggaagctgc tcacccaaga tttggtgcag gaaaagtacc tggagtaccg 1380
   gcaggtgccg gacagtgatc ccgcacgcta tgagttcctg tggggtccaa gggccctcgc 1440
   tgaaaccagc tatgtgaaag tccttgagta tgtgatcaag gtcagtgcaa gagttcgctt 1500
   tttcttccca tccctgcgtg aagcagcttt gagagaggag gaagagggag tctgagcatg 1560
   agttgcagcc aaggccagtg ggagggggac tgggccagtg caccttccag ggccgcgtcc 1620
   agcagcttcc cctgcctcgt gtgacatgag gcccattctt cactctgaag agagcggtca 1680
   gtgttctcag tagtaggttt ctgttctatt gggtgacttg gagatttatc tttgttctct 1740
   tttggaattg ttcaaatgtt tttttttaag ggatggttga atgaacttca gcatccaagt 1800
   ttatgaatga cagcagtcac acagttctgt gtatatagtt taagggtaag agtcttgtgt 1860
   tttattcaga ttgggaaatc cattctattt tgtgaattgg gataataaca gcagtggaat 1920
   aagtacttag aaatgtgaaa aatgagcagt aaaatagatg agataaagaa ctaaagaaat 1980
   taagagatag tcaattcttg ccttatacct cagtctattc tgtaaaattt ttaaagatat 2040
   atgcatacct ggatttcctt ggcttctttg agaatgtaag agaaattaaa tctgaataaa 2100
   gaattcttcc tgttcactgg ctcttttctt ctccatgcac tgagcatctg ctttttggaa 2160
   ggccctgggt tagtagtgga gatgctaagg taagccagac tcatacccac ccatagggtc 2220
   gtagagtcta ggagctgcag tcacgtaatc gaggtggcaa gatgtcctct aaagatgtag 2280
   ggaaaagtga gagaggggtg agggtgtggg gctccgggtg agagtggtgg agtgtcaatg 2340
   ccctgagctg gggcattttg ggctttggga aactgcagtt ccttctgggg gagctgattg 2400
   taatgatctt gggtggatcc                                             2420
<210> 19
   <211> 4204
   <212> DNA
   <213> Homo sapiens
<400> 19
<210> 20
   <211> 752
   <212> DNA
   <213> Homo sapiens
<400> 20
   atcctcgtgg gccctgacct tctctctgag agccgggcag aggctccgga gccatgcagg 60
   ccgaaggccg gggcacaggg ggttcgacgg gcgatgctga tggcccagga ggccctggca 120
   ttcctgatgg cccagggggc aatgctggcg gcccaggaga ggcgggtgcc acgggcggca 180
   gaggtccccg gggcgcaggg gcagcaaggg cctcggggcc gggaggaggc gccccgcggg 240
   gtccgcatgg cggcgcggct tcagggctga atggatgctg cagatgcggg gccagggggc 300
   cggagagccg cctgcttgag ttctacctcg ccatgccttt cgcgacaccc atggaagcag 360
   agctggcccg caggagcctg gcccaggatg ccccaccgct tcccgtgcca ggggtgcttc 420
   tgaaggagtt cactgtgtcc ggcaacatac tgactatccg actgactgct gcagaccacc 480
   gccaactgca gctctccatc agctcctgtc tccagcagct ttccctgttg atgtggatca 540
   cgcagtgctt tctgcccgtg tttttggctc agcctccctc agggcagagg cgctaagccc 600
   agcctggcgc cccttcctag gtcatgcctc ctcccctagg gaatggtccc agcacgagtg 660
   gccagttcat tgtgggggcc tgattgtttg tcgctggagg aggacggctt acatgtttgt 720
   ttctgtagaa aataaaactg agctacgaaa aa                               752
<210> 21
   <211> 2148
   <212> DNA
   <213> Homo sapiens
<400> 21
   gcttcagggt acagctcccc cgcagccaga agccgggcct gcagcgcctc agcaccgctc 60
   cgggacaccc cacccgcttc ccaggcgtga cctgtcaaca gcaacttcgc ggtgtggtga 120
   actctctgag gaaaaaccat tttgattatt actctcagac gtgcgtggca acaagtgact 180
   gagacctaga aatccaagcg ttggaggtcc tgaggccagc ctaagtcgct tcaaaatgga 240
   acgaaggcgt ttgtggggtt ccattcagag ccgatacatc agcatgagtg tgtggacaag 300
   cccacggaga cttgtggagc tggcagggca gagcctgctg aaggatgagg ccctggccat 360
   tgccgccctg gagttgctgc ccagggagct cttcccgcca ctcttcatgg cagcctttga 420
   cgggagacac agccagaccc tgaaggcaat ggtgcaggcc tggcccttca cctgcctccc 480
   tctgggagtg ctgatgaagg gacaacatct tcacctggag accttcaaag ctgtgcttga 540
   tggacttgat gtgctccttg cccaggaggt tcgccccagg aggtggaaac ttcaagtgct 600
   ggatttacgg aagaactctc atcaggactt ctggactgta tggtctggaa acagggccag 660
   tctgtactca tttccagagc cagaagcagc tcagcccatg acaaagaagc gaaaagtaga 720
   tggtttgagc acagaggcag agcagccctt cattccagta gaggtgctcg tagacctgtt 780
   cctcaaggaa ggtgcctgtg atgaattgtt ctcctacctc attgagaaag tgaagcgaaa 840
   gaaaaatgta ctacgcctgt gctgtaagaa gctgaagatt tttgcaatgc ccatgcagga 900
   tatcaagatg atcctgaaaa tggtgcagct ggactctatt gaagatttgg aagtgacttg 960
   tacctggaag ctacccacct tggcgaaatt ttctccttac ctgggccaga tgattaatct 1020
   gcgtagactc ctcctctccc acatccatgc atcttcctac atttccccgg agaaggaaga 1080
   gcagtatatc gcccagttca cctctcagtt cctcagtctg cagtgcctgc aggctctcta 1140
   tgtggactct ttatttttcc ttagaggccg cctggatcag ttgctcaggc acgtgatgaa 1200
   ccccttggaa accctctcaa taactaactg ccggctttcg gaaggggatg tgatgcatct 1260
   gtcccagagt cccagcgtca gtcagctaag tgtcctgagt ctaagtgggg tcatgctgac 1320
   cgatgtaagt cccgagcccc tccaagctct gctggagaga gcctctgcca ccctccagga 1380
   cctggtcttt gatgagtgtg ggatcacgga tgatcagctc cttgccctcc tgccttccct 1440
   gagccactgc tcccagctta caaccttaag cttctacggg aattccatct ccatatctgc 1500
   cttgcagagt ctcctgcagc acctcatcgg gctgagcaat ctgacccacg tgctgtatcc 1560
   tgtccccctg gagagttatg aggacatcca tggtaccctc cacctggaga ggcttgccta 1620
   tctgcatgcc aggctcaggg agttgctgtg tgagttgggg cggcccagca tggtctggct 1680
   tagtgccaac ccctgtcctc actgtgggga cagaaccttc tatgacccgg agcccatcct 1740
   gtgcccctgt ttcatgccta actagctggg tgcacatatc aaatgcttca ttctgcatac 1800
   ttggacacta aagccaggat gtgcatgcat cttgaagcaa caaagcagcc acagtttcag 1860
   acaaatgttc agtgtgagtg aggaaaacat gttcagtgag gaaaaaacat tcagacaaat 1920
   gttcagtgag gaaaaaaagg ggaagttggg gataggcaga tgttgacttg aggagttaat 1980
   gtgatctttg gggagataca tcttatagag ttagaaatag aatctgaatt tctaaaggga 2040
   gattctggct tgggaagtac atgtaggagt taatccctgt gtagactgtt gtaaagaaac 2100
   tgttgaaaat aaagagaagc aatgtgaagc aaaaaaaaaa aaaaaaaa               2148
<210> 22
   <211> 4530
   <212> DNA
   <213> Homo sapiens
<400> 22
   aattctcgag ctcgtcgacc ggtcgacgag ctcgagggtc gacgagctcg agggcgcgcg 60
   cccggccccc acccctcgca gcaccccgcg ccccgcgccc tcccagccgg gtccagccgg 120
   agccatgggg ccggagccgc agtgagcacc atggagctgg cggccttgtg ccgctggggg 180
   ctcctcctcg ccctcttgcc ccccggagcc gcgagcaccc aagtgtgcac cggcacagac 240
   atgaagctgc ggctccctgc cagtcccgag acccacctgg acatgctccg ccacctctac 300
   cagggctgcc aggtggtgca gggaaacctg gaactcacct acctgcccac caatgccagc 360
   ctgtccttcc tgcaggatat ccaggaggtg cagggctacg tgctcatcgc tcacaaccaa 420
   gtgaggcagg tcccactgca gaggctgcgg attgtgcgag gcacccagct ctttgaggac 480
   aactatgccc tggccgtgct agacaatgga gacccgctga acaataccac ccctgtcaca 540
   ggggcctccc caggaggcct gcgggagctg cagcttcgaa gcctcacaga gatcttgaaa 600
   ggaggggtct tgatccagcg gaacccccag ctctgctacc aggacacgat tttgtggaag 660
   gacatcttcc acaagaacaa ccagctggct ctcacactga tagacaccaa ccgctctcgg 720
   gcctgccacc cctgttctcc gatgtgtaag ggctcccgct gctggggaga gagttctgag 780
   gattgtcaga gcctgacgcg cactgtctgt gccggtggct gtgcccgctg caaggggcca 840
   ctgcccactg actgctgcca tgagcagtgt gctgccggct gcacgggccc caagcactct 900
   gactgcctgg cctgcctcca cttcaaccac agtggcatct gtgagctgca ctgcccagcc 960
   ctggtcacct acaacacaga cacgtttgag tccatgccca atcccgaggg ccggtataca 1020
   ttcggcgcca gctgtgtgac tgcctgtccc tacaactacc tttctacgga cgtgggatcc 1080
   tgcaccctcg tctgccccct gcacaaccaa gaggtgacag cagaggatgg aacacagcgg 1140
   tgtgagaagt gcagcaagcc ctgtgcccga gtgtgctatg gtctgggcat ggagcacttg 1200
   cgagaggtga gggcagttac cagtgccaat atccaggagt ttgctggctg caagaagatc 1260
   tttgggagcc tggcatttct gccggagagc tttgatgggg acccagcctc caacactgcc 1320
   ccgctccagc cagagcagct ccaagtgttt gagactctgg aagagatcac aggttaccta 1380
   tacatctcag catggccgga cagcctgcct gacctcagcg tcttccagaa cctgcaagta 1440
   atccggggac gaattctgca caatggcgcc tactcgctga ccctgcaagg gctgggcatc 1500
   agctggctgg ggctgcgctc actgagggaa ctgggcagtg gactggccct catccaccat 1560
   aacacccacc tctgcttcgt gcacacggtg ccctgggacc agctctttcg gaacccgcac 1620
   caagctctgc tccacactgc caaccggcca gaggacgagt gtgtgggcga gggcctggcc 1680
   tgccaccagc tgtgcgcccg agggcactgc tggggtccag ggcccaccca gtgtgtcaac 1740
   tgcagccagt tccttcgggg ccaggagtgc gtggaggaat gccgagtact gcaggggctc 1800
   cccagggagt atgtgaatgc caggcactgt ttgccgtgcc accctgagtg tcagccccag 1860
   aatggctcag tgacctgttt tggaccggag gctgaccagt gtgtggcctg tgcccactat 1920
   aaggaccctc ccttctgcgt ggcccgctgc cccagcggtg tgaaacctga cctctcctac 1980
   atgcccatct ggaagtttcc agatgaggag ggcgcatgcc agccttgccc catcaactgc 2040
   acccactcct gtgtggacct ggatgacaag ggctgccccg ccgagcagag agccagccct 2100
   ctgacgtcca tcgtctctgc ggtggttggc attctgctgg tcgtggtctt gggggtggtc 2160
   tttgggatcc tcatcaagcg acggcagcag aagatccgga agtacacgat gcggagactg 2220
   ctgcaggaaa cggagctggt ggagccgctg acacctagcg gagcgatgcc caaccaggcg 2280
   cagatgcgga tcctgaaaga gacggagctg aggaaggtga aggtgcttgg atctggcgct 2340
   tttggcacag tctacaaggg catctggatc cctgatgggg agaatgtgaa aattccagtg 2400
   gccatcaaag tgttgaggga aaacacatcc cccaaagcca acaaagaaat cttagacgaa 2460
   gcatacgtga tggctggtgt gggctcccca tatgtctccc gccttctggg catctgcctg 2520
   acatccacgg tgcagctggt gacacagctt atgccctatg gctgcctctt agaccatgtc 2580
   cgggaaaacc gcggacgcct gggctcccag gacctgctga actggtgtat gcagattgcc 2640
   aaggggatga gctacctgga ggatgtgcgg ctcgtacaca gggacttggc cgctcggaac 2700
   gtgctggtca agagtcccaa ccatgtcaaa attacagact tcgggctggc tcggctgctg 2760
   gacattgacg agacagagta ccatgcagat gggggcaagg tgcccatcaa gtggatggcg 2820
   ctggagtcca ttctccgccg gcggttcacc caccagagtg atgtgtggag ttatggtgtg 2880
   actgtgtggg agctgatgac ttttggggcc aaaccttacg atgggatccc agcccgggag 2940
   atccctgacc tgctggaaaa gggggagcgg ctgccccagc cccccatctg caccattgat 3000
   gtctacatga tcatggtcaa atgttggatg attgactctg aatgtcggcc aagattccgg 3060
   gagttggtgt ctgaattctc ccgcatggcc agggaccccc agcgctttgt ggtcatccag 3120
   aatgaggact tgggcccagc cagtcccttg gacagcacct tctaccgctc actgctggag 3180
   gacgatgaca tgggggacct ggtggatgct gaggagtatc tggtacccca gcagggcttc 3240
   ttctgtccag accctgcccc gggcgctggg ggcatggtcc accacaggca ccgcagctca 3300
   tctaccagga gtggcggtgg ggacctgaca ctagggctgg agccctctga agaggaggcc 3360
   cccaggtctc cactggcacc ctccgaaggg gctggctccg atgtatttga tggtgacctg 3420
   ggaatggggg cagccaaggg gctgcaaagc ctccccacac atgaccccag ccctctacag 3480
   cggtacagtg aggaccccac agtacccctg ccctctgaga ctgatggcta cgttgccccc 3540
   ctgacctgca gcccccagcc tgaatatgtg aaccagccag atgttcggcc ccagccccct 3600
   tcgccccgag agggccctct gcctgctgcc cgacctgctg gtgccactct ggaaagggcc 3660
   aagactctct ccccagggaa gaatggggtc gtcaaagacg tttttgcctt tgggggtgcc 3720
   gtggagaacc ccgagtactt gacaccccag ggaggagctg cccctcagcc ccaccctcct 3780
   cctgccttca gcccagcctt cgacaacctc tattactggg accaggaccc accagagcgg 3840
   ggggctccac ccagcacctt caaagggaca cctacggcag agaacccaga gtacctgggt 3900
   ctggacgtgc cagtgtgaac cagaaggcca agtccgcaga agccctgatg tgtcctcagg 3960
   gagcagggaa ggcctgactt ctgctggcat caagaggtgg gagggccctc cgaccacttc 4020
   caggggaacc tgccatgcca ggaacctgtc ctaaggaacc ttccttcctg cttgagttcc 4080
   cagatggctg gaaggggtcc agcctcgttg gaagaggaac agcactgggg agtctttgtg 4140
   gattctgagg ccctgcccaa tgagactcta gggtccagtg gatgccacag cccagcttgg 4200
   ccctttcctt ccagatcctg ggtactgaaa gccttaggga agctggcctg agaggggaag 4260
   cggccctaag ggagtgtcta agaacaaaag cgacccattc agagactgtc cctgaaacct 4320
   agtactgccc cccatgagga aggaacagca atggtgtcag tatccaggct ttgtacagag 4380
   tgcttttctg tttagttttt actttttttg ttttgttttt ttaaagacga aataaagacc 4440
   caggggagaa tgggtgttgt atggggaggc aagtgtgggg ggtccttctc cacacccact 4500
   ttgtccattt gcaaatatat tttggaaaac                                  4530
<210> 23
   <211> 2388
   <212> DNA
   <213> Homo sapiens
<400> 23
   ggaaaccgag gcagaggagg ctcaggtgtg gccaatcacc ctgcacatca gagttaccct 60
   gggcagggcc cactgagacc tgggaggggc cactcgggac ctggagggct gggggctgcc 120
   cgggcgttag gggtaaagct ccctacccaa ctgcgcagaa ggcctcagag gcctgggggc 180
   tgggcttccc ctttcacatc gccctttaga ggcccacgtg tgggcattgg cccgcgatct 240
   gaaaggggct gtcctgttcc tcatgggcgc tgccagcgcc acgcactcct ctttctgcct 300
   ggccggccac tcccgtctgc tgtgacgcgc ggacagagag ctaccggtgg acccacggtg 360
   cctccctccc tgggatctac acagaccatg gccttgccaa cggctcgacc cctgttgggg 420
   tcctgtggga cccccgccct cggcagcctc ctgttcctgc tcttcagcct cggatgggtg 480
   cagccctcga ggaccctggc tggagagaca gggcaggagg ctgcgcccct ggacggagtc 540
   ctggccaacc cacctaacat ttccagcctc tcccctcgcc aactccttgg cttcccgtgt 600
   gcggaggtgt ccggcctgag cacggagcgt gtccgggagc tggctgtggc cttggcacag 660
   aagaatgtca agctctcaac agagcagctg cgctgtctgg ctcaccggct ctctgagccc 720
   cccgaggacc tggacgccct cccattggac ctgctgctat tcctcaaccc agatgcgttc 780
   tcggggcccc aggcctgcac ccgtttcttc tcccgcatca cgaaggccaa tgtggacctg 840
   ctcccgaggg gggctcccga gcgacagcgg ctgctgcctg cggctctggc ctgctggggt 900
   gtgcgggggt ctctgctgag cgaggctgat gtgcgggctc tgggaggcct ggcttgcgac 960
   ctgcctgggc gctttgtggc cgagtcggcc gaagtgctgc taccccggct ggtgagctgc 1020
   ccgggacccc tggaccagga ccagcaggag gcagccaggg cggctctgca gggcggggga 1080
   cccccctacg gccccccgtc gacatggtct gtctccacga tggacgctct gcggggcctg 1140
   ctgcccgtgc tgggccagcc catcatccgc agcatcccgc agggcatcgt ggccgcgtgg 1200
   cggcaacgct cctctcggga cccatcctgg cggcagcctg aacggaccat cctccggccg 1260
   cggttccggc gggaagtgga gaagacagcc tgtccttcag gcaagaaggc ccgcgagata 1320
   gacgagagcc tcatcttcta caagaagtgg gagctggaag cctgcgtgga tgcggccctg 1380
   ctggccaccc agatggaccg cgtgaacgcc atccccttca cctacgagca gctggacgtc 1440
   ctaaagcata aactggatga gctctaccca caaggttacc ccgagtctgt gatccagcac 1500
   ctgggctacc tcttcctcaa gatgagccct gaggacattc gcaagtggaa tgtgacgtcc 1560
   ctggagaccc tgaaggcttt gcttgaagtc aacaaagggc acgaaatgag tcctcaggtg 1620
   gccaccctga tcgaccgctt tgtgaaggga aggggccagc tagacaaaga caccctagac 1680
   accctgaccg ccttctaccc tgggtacctg tgctccctca gccccgagga gctgagctcc 1740
   gtgcccccca gcagcatctg ggcggtcagg ccccaggacc tggacacgtg tgacccaagg 1800
   cagctggacg tcctctatcc caaggcccgc cttgctttcc agaacatgaa cgggtccgaa 1860
   tacttcgtga agatccagtc cttcctgggt ggggccccca cggaggattt gaaggcgctc 1920
   agtcagcaga atgtgagcat ggacttggcc acgttcatga agctgcggac ggatgcggtg 1980
   ctgccgttga ctgtggctga ggtgcagaaa cttctgggac cccacgtgga gggcctgaag 2040
   gcggaggagc ggcaccgccc ggtgcgggac tggatcctac ggcagcggca ggacgacctg 2100
   gacacgctgg ggctggggct acagggcggc atccccaacg gctacctggt cctagacctc 2160
   agcatgcaag aggccctctc ggggacgccc tgcctcctag gacctggacc tgttctcacc 2220
   gtcctggcac tgctcctagc ctccaccctg gcctgagggc cccactccct tgctggcccc 2280
   agccctgctg gggatccccg cctggccagg agcaggcacg ggtggtcccc gttccacccc 2340
   aagagaactc gcgctcagta aacgggaaca tgccccctgc agacacgt 2388
<210> 24
   <211> 2412
   <212> DNA
   <213> Homo sapiens
<400> 24
   ggaaaccgag gcagaggagg ctcaggtgtg gccaatcacc ctgcacatca gagttaccct 60
   gggcagggcc cactgagacc tgggaggggc cactcgggac ctggagggct gggggctgcc 120
   cgggcgttag gggtaaagct ccctacccaa ctgcgcagaa ggcctcagag gcctgggggc 180
   tgggcttccc ctttcacatc gccctttaga ggcccacgtg tgggcattgg cccgcgatct 240
   gaaaggggct gtcctgttcc tcatgggcgc tgccagcgcc acgcactcct ctttctgcct 300
   ggccggccac tcccgtctgc tgtgacgcgc ggacagagag ctaccggtgg acccacggtg 360
   cctccctccc tgggatctac acagaccatg gccttgccaa cggctcgacc cctgttgggg 420
   tcctgtggga cccccgccct cggcagcctc ctgttcctgc tcttcagcct cggatgggtg 480
   cagccctcga ggaccctggc tggagagaca gggcaggagg ctgcgcccct ggacggagtc 540
   ctggccaacc cacctaacat ttccagcctc tcccctcgcc aactccttgg cttcccgtgt 600
   gcggaggtgt ccggcctgag cacggagcgt gtccgggagc tggctgtggc cttggcacag 660
   aagaatgtca agctctcaac agagcagctg cgctgtctgg ctcaccggct ctctgagccc 720
   cccgaggacc tggacgccct cccattggac ctgctgctat tcctcaaccc agatgcgttc 780
   tcggggcccc aggcctgcac ccgtttcttc tcccgcatca cgaaggccaa tgtggacctg 840
   ctcccgaggg gggctcccga gcgacagcgg ctgctgcctg cggctctggc ctgctggggt 900
   gtgcgggggt ctctgctgag cgaggctgat gtgcgggctc tgggaggcct ggcttgcgac 960
   ctgcctgggc gctttgtggc cgagtcggcc gaagtgctgc taccccggct ggtgagctgc 1020
   ccgggacccc tggaccagga ccagcaggag gcagccaggg cggctctgca gggcggggga 1080
   cccccctacg gccccccgtc gacatggtct gtctccacga tggacgctct gcggggcctg 1140
   ctgcccgtgc tgggccagcc catcatccgc agcatcccgc agggcatcgt ggccgcgtgg 1200
   cggcaacgct cctctcggga cccatcctgg cggcagcctg aacggaccat cctccggccg 1260
   cggttccggc gggaagtgga gaagacagcc tgtccttcag gcaagaaggc ccgcgagata 1320
   gacgagagcc tcatcttcta caagaagtgg gagctggaag cctgcgtgga tgcggccctg 1380
   ctggccaccc agatggaccg cgtgaacgcc atccccttca cctacgagca gctggacgtc 1440
   ctaaagcata aactggatga gctctaccca caaggttacc ccgagtctgt gatccagcac 1500
   ctgggctacc tcttcctcaa gatgagccct gaggacattc gcaagtggaa tgtgacgtcc 1560
   ctggagaccc tgaaggcttt gcttgaagtc aacaaagggc acgaaatgag tcctcaggct 1620
   cctcggcggc ccctcccaca ggtggccacc ctgatcgacc gctttgtgaa gggaaggggc 1680
   cagctagaca aagacaccct agacaccctg accgccttct accctgggta cctgtgctcc 1740
   ctcagccccg aggagctgag ctccgtgccc cccagcagca tctgggcggt caggccccag 1800
   gacctggaca cgtgtgaccc aaggcagctg gacgtcctct atcccaaggc ccgccttgct 1860
   ttccagaaca tgaacgggtc cgaatacttc gtgaagatcc agtccttcct gggtggggcc 1920
   cccacggagg atttgaaggc gctcagtcag cagaatgtga gcatggactt ggccacgttc 1980
   atgaagctgc ggacggatgc ggtgctgccg ttgactgtgg ctgaggtgca gaaacttctg 2040
   ggaccccacg tggagggcct gaaggcggag gagcggcacc gcccggtgcg ggactggatc 2100
   ctacggcagc ggcaggacga cctggacacg ctggggctgg ggctacaggg cggcatcccc 2160
   aacggctacc tggtcctaga cctcagcatg caagaggccc tctcggggac gccctgcctc 2220
   ctaggacctg gacctgttct caccgtcctg gcactgctcc tagcctccac cctggcctga 2280
   gggccccact cccttgctgg ccccagccct gctggggatc cccgcctggc caggagcagg 2340
   cacgggtggt ccccgttcca ccccaagaga actcgcgctc agtaaacggg aacatgcccc 2400
   ctgcagacac gt                                                    2412
<210> 25
   <211> 3665
   <212> DNA
   <213> Homo sapiens
<400> 25
   ggcttggggc agccgggtag ctcggaggtc gtggcgctgg gggctagcac cagcgctctg 60
   tcgggaggcg cagcggttag gtggaccggt cagcggactc accggccagg gcgctcggtg 120
   ctggaatttg atattcattg atccgggttt tatccctctt cttttttctt aaacattttt 180
   ttttaaaact gtattgtttc tcgttttaat ttatttttgc ttgccattcc ccacttgaat 240
   cgggccgacg gcttggggag attgctctac ttccccaaat cactgtggat tttggaaacc 300
   agcagaaaga ggaaagaggt agcaagagct ccagagagaa gtcgaggaag agagagacgg 360
   ggtcagagag agcgcgcggg cgtgcgagca gcgaaagcga caggggcaaa gtgagtgacc 420
   tgcttttggg ggtgaccgcc ggagcgcggc gtgagccctc ccccttggga tcccgcagct 480
   gaccagtcgc gctgacggac agacagacag acaccgcccc cagccccagc taccacctcc 540
   tccccggccg gcggcggaca gtggacgcgg cggcgagccg cgggcagggg ccggagcccg 600
   cgcccggagg cggggtggag ggggtcgggg ctcgcggcgt cgcactgaaa cttttcgtcc 660
   aacttctggg ctgttctcgc ttcggaggag ccgtggtccg cgcgggggaa gccgagccga 720
   gcggagccgc gagaagtgct agctcgggcc gggaggagcc gcagccggag gagggggagg 780
   aggaagaaga gaaggaagag gagagggggc cgcagtggcg actcggcgct cggaagccgg 840
   gctcatggac gggtgaggcg gcggtgtgcg cagacagtgc tccagccgcg cgcgctcccc 900
   aggccctggc ccgggcctcg ggccggggag gaagagtagc tcgccgaggc gccgaggaga 960
   gcgggccgcc ccacagcccg agccggagag ggagcgcgag ccgcgccggc cccggtcggg 1020
   cctccgaaac catgaacttt ctgctgtctt gggtgcattg gagccttgcc ttgctgctct 1080
   acctccacca tgccaagtgg tcccaggctg cacccatggc agaaggagga gggcagaatc 1140
   atcacgaagt ggtgaagttc atggatgtct atcagcgcag ctactgccat ccaatcgaga 1200
   ccctggtgga catcttccag gagtaccctg atgagatcga gtacatcttc aagccatcct 1260
   gtgtgcccct gatgcgatgc gggggctgct gcaatgacga gggcctggag tgtgtgccca 1320
   ctgaggagtc caacatcacc atgcagatta tgcggatcaa acctcaccaa ggccagcaca 1380
   taggagagat gagcttccta cagcacaaca aatgtgaatg cagaccaaag aaagatagag 1440
   caagacaaga aaaaaaatca gttcgaggaa agggaaaggg gcaaaaacga aagcgcaaga 1500
   aatcccggta taagtcctgg agcgtgtacg ttggtgcccg ctgctgtcta atgccctgga 1560
   gcctccctgg cccccatccc tgtgggcctt gctcagagcg gagaaagcat ttgtttgtac 1620
   aagatccgca gacgtgtaaa tgttcctgca aaaacacaga ctcgcgttgc aaggcgaggc 1680
   agcttgagtt aaacgaacgt acttgcagat gtgacaagcc gaggcggtga gccgggcagg 1740
   aggaaggagc ctccctcagg gtttcgggaa ccagatctct caccaggaaa gactgataca 1800
   gaacgatcga tacagaaacc acgctgccgc caccacacca tcaccatcga cagaacagtc 1860
   cttaatccag aaacctgaaa tgaaggaaga ggagactctg cgcagagcac tttgggtccg 1920
   gagggcgaga ctccggcgga agcattcccg ggcgggtgac ccagcacggt ccctcttgga 1980
   attggattcg ccattttatt tttcttgctg ctaaatcacc gagcccggaa gattagagag 2040
   ttttatttct gggattcctg tagacacacc cacccacata catacattta tatatatata 2100
   tattatatat atataaaaat aaatatctct attttatata tataaaatat atatattctt 2160
   tttttaaatt aacagtgcta atgttattgg tgtcttcact ggatgtattt gactgctgtg 2220
   gacttgagtt gggaggggaa tgttcccact cagatcctga cagggaagag gaggagatga 2280
   gagactctgg catgatcttt tttttgtccc acttggtggg gccagggtcc tctcccctgc 2340
   ccaggaatgt gcaaggccag ggcatggggg caaatatgac ccagttttgg gaacaccgac 2400
   aaacccagcc ctggcgctga gcctctctac cccaggtcag acggacagaa agacagatca 2460
   caggtacagg gatgaggaca ccggctctga ccaggagttt ggggagcttc aggacattgc 2520
   tgtgctttgg ggattccctc cacatgctgc acgcgcatct cgcccccagg ggcactgcct 2580
   ggaagattca ggagcctggg cggccttcgc ttactctcac ctgcttctga gttgcccagg 2640
   agaccactgg cagatgtccc ggcgaagaga agagacacat tgttggaaga agcagcccat 2700
   gacagctccc cttcctggga ctcgccctca tcctcttcct gctccccttc ctggggtgca 2760
   gcctaaaagg acctatgtcc tcacaccatt gaaaccacta gttctgtccc cccaggagac 2820
   ctggttgtgt gtgtgtgagt ggttgacctt cctccatccc ctggtccttc ccttcccttc 2880
   ccgaggcaca gagagacagg gcaggatcca cgtgcccatt gtggaggcag agaaaagaga 2940
   aagtgtttta tatacggtac ttatttaata tcccttttta attagaaatt aaaacagtta 3000
   atttaattaa agagtagggt tttttttcag tattcttggt taatatttaa tttcaactat 3060
   ttatgagatg tatcttttgc tctctcttgc tctcttattt gtaccggttt ttgtatataa 3120
   aattcatgtt tccaatctct ctctccctga tcggtgacag tcactagctt atcttgaaca 3180
   gatatttaat tttgctaaca ctcagctctg ccctccccga tcccctggct ccccagcaca 3240
   cattcctttg aaataaggtt tcaatataca tctacatact atatatatat ttggcaactt 3300
   gtatttgtgt gtatatatat atatatatgt ttatgtatat atgtgattct gataaaatag 3360
   acattgctat tctgtttttt atatgtaaaa acaaaacaag aaaaaataga gaattctaca 3420
   tactaaatct ctctcctttt ttaattttaa tatttgttat catttattta ttggtgctac 3480
   tgtttatccg taataattgt ggggaaaaga tattaacatc acgtctttgt ctctagtgca 3540
   gtttttcgag atattccgta gtacatattt atttttaaac aacgacaaag aaatacagat 3600
   atatcttaaa aaaaaaaaag cattttgtat taaagaattt aattctgatc tcaaaaaaaa 3660
   aaaaa 3665
<210> 26
   <211> 2204
   <212> DNA
   <213> Homo sapiens
<400> 26
   gagcggtgcg gaggctctgc tcggatcgag gtctgcagcg cagcttcggg agcatgagtg 60
   ctgcagtgac tgcagggaag ctggcacggg caccggccga ccctgggaaa gccggggtcc 120
   ccggagttgc agctcccgga gctccggcgg cggctccacc ggcgaaagag atcccggagg 180
   tcctagtgga cccacgcagc cggcggcgct atgtgcgggg ccgctttttg ggcaagggcg 240
   gctttgccaa gtgcttcgag atctcggacg cggacaccaa ggaggtgttc gcgggcaaga 300
   ttgtgcctaa gtctctgctg ctcaagccgc accagaggga gaagatgtcc atggaaatat 360
   ccattcaccg cagcctcgcc caccagcacg tcgtaggatt ccacggcttt ttcgaggaca 420
   acgacttcgt gttcgtggtg ttggagctct gccgccggag gtctctcctg gagctgcaca 480
   agaggaggaa agccctgact gagcctgagg cccgatacta cctacggcaa attgtgcttg 540
   gctgccagta cctgcaccga aaccgagtta ttcatcgaga cctcaagctg ggcaaccttt 600
   tcctgaatga agatctggag gtgaaaatag gggattttgg actggcaacc aaagtcgaat 660
   atgacgggga gaggaagaag accctgtgtg ggactcctaa ttacatagct cccgaggtgc 720
   tgagcaagaa agggcacagt ttcgaggtgg atgtgtggtc cattgggtgt atcatgtata 780
   ccttgttagt gggcaaacca ccttttgaga cttcttgcct aaaagagacc tacctccgga 840
   tcaagaagaa tgaatacagt attcccaagc acatcaaccc cgtggccgcc tccctcatcc 900
   agaagatgct tcagacagat cccactgccc gcccaaccat taacgagctg cttaatgacg 960
   agttctttac ttctggctat atccctgccc gtctccccat cacctgcctg accattccac 1020
   caaggttttc gattgctccc agcagcctgg accccagcaa ccggaagccc ctcacagtcc 1080
   tcaataaagg cttggagaac cccctgcctg agcgtccccg ggaaaaagaa gaaccagtgg 1140
   ttcgagagac aggtgaggtg gtcgactgcc acctcagtga catgctgcag cagctgcaca 1200
   gtgtcaatgc ctccaagccc tcggagcgtg ggctggtcag gcaagaggag gctgaggatc 1260
   ctgcctgcat ccccatcttc tgggtcagca agtgggtgga ctattcggac aagtacggcc 1320
   ttgggtatca gctctgtgat aacagcgtgg gggtgctctt caatgactca acacgcctca 1380
   tcctctacaa tgatggtgac agcctgcagt acatagagcg tgacggcact gagtcctacc 1440
   tcaccgtgag ttcccatccc aactccttga tgaagaagat caccctcctt aaatatttcc 1500
   gcaattacat gagcgagcac ttgctgaagg caggtgccaa catcacgccg cgcgaaggtg 1560
   atgagctcgc ccggctgccc tacctacgga cctggttccg cacccgcagc gccatcatcc 1620
   tgcacctcag caacggcagc gtgcagatca acttcttcca ggatcacacc aagctcatct 1680
   tgtgcccact gatggcagcc gtgacctaca tcgacgagaa gcgggacttc cgcacatacc 1740
   gcctgagtct cctggaggag tacggctgct gcaaggagct ggccagccgg ctccgctacg 1800
   cccgcactat ggtggacaag ctgctgagct cacgctcggc cagcaaccgt ctcaaggcct 1860
   cctaatagct gccctcccct ccggactggt gccctcctca ctcccacctg catctggggc 1920
   ccatactggt tggctcccgc ggtgccatgt ctgcagtgtg ccccccagcc ccggtggctg 1980
   ggcagagctg catcatcctt gcaggtgggg gttgctgtgt aagttatttt tgtacatgtt 2040
   cgggtgtggg ttctacagcc ttgtccccct ccccctcaac cccaccatat gaattgtaca 2100
   gaatatttct attgaattcg gaactgtcct ttccttggct ttatgcacat taaacagatg 2160
   tgaatattca aaaaaaaaaa aaaaaaaaaa aaaaaaaaaa aaaa 2204